# EUROPEAN PATENT APPLICATION

(11) **EP 4 116 713 A2**
(43) Date of publication of application: **11.01.2023**
(21) Application number: 22173266.2
(22) Date of filing: 10.09.2016
(51) Int. Cl.: G01N 33/564, G01N 33/536, G01N 33/543

(54) **COMPOSITIONS, DEVICES, AND METHODS OF OSTEOARTHRITIS SENSITIVITY TESTING**

(30) Priority: 09.09.2015 US 201562216272 P
(62) Divisional of application: 16845236.5
(71) Applicant: Biomerica, Inc., Irvine, California 92614 (US)
(72) Inventor: IRANI-COHEN, Zackary, Irvine, CA 92614 (US); LADERMAN, Elisabeth, Irvine, CA 92614 (US)
(74) Representative: Wright, Simon Mark

(57) **Abstract**

Contemplated test kits and methods for food sensitivity related to osteoarthritis are based on rational-based selection of food preparations with established discriminatory p-value. In some embodiments, kits include those with a minimum number of food preparations that have an average discriminatory p-value of ≤ 0.07 as determined by their raw p-value or an average discriminatory p-value of ≤ 0.10 as determined by FDR multiplicity adjusted p-value. In further contemplated aspects, compositions and methods for food sensitivity are also stratified by gender to further enhance predictive value.

## Description

This application claims priority to our U.S. provisional patent application with the serial number 62/216,272 filed September 9, 2015, which is incorporated by reference herein in its entirety.

### Field

The field of the subject matter disclosed herein is sensitivity testing for food intolerance, and especially as it relates to testing and possible elimination of selected food items as foods that exacerbate or worsen symptoms or foods that, when removed, alleviate symptoms in patients diagnosed with or suspected to have osteoarthritis.

### Background

The background description includes information that may be useful in understanding the present disclosure. It is not an admission that any of the information provided herein is prior art or relevant to the disclosure, or that any publication specifically or implicitly referenced is prior art.

Food sensitivity, especially as it relates to osteoarthritis (a type of inflammatory disorder), often presents with joint pain, stiffness, joint swelling, decreased range of motion, and numbness in the arms and legs and underlying causes of osteoarthritis are not well understood in the medical community. Most typically, osteoarthritis is diagnosed by medical imaging and other tests, which are occasionally used to either support or rule out other problems. While exercise along with some medications or joint surgery are recommended to treat osteoarthritis, unfortunately, there are no medications that directly treat the core symptoms of osteoarthritis. Elimination of either one or more food items has also shown promise in at least reducing incidence and/or severity of the symptoms. However, osteoarthritis is often quite diverse with respect to dietary items triggering or exacerbating symptoms, and no standardized test to help identify trigger food items that exacerbate or worsen symptoms or whose removal results in alleviation of symptoms with a reasonable degree of certainty is known, leaving affected patients often to trial-and-error.

While there are some commercially available tests and labs to help identify trigger foods for food allergies, no commercially available tests are specifically directed to test food allergens in association with osteoarthritis. Furthermore, the quality of the test results from these labs is generally poor as is reported by a consumer advocacy group (e.g., http://www.which.co.uk /news/2008/08/food-allergy-tests-could-risk-your-health-154711/). Most notably, problems associated with these tests and labs were high false positive rates, high false negative rates, high intra-patient variability, and high inter-laboratory variability, rendering such tests nearly useless. Similarly, further inconclusive and highly variable test results were also reported elsewhere (Alternative Medicine Review, Vol. 9, No. 2, 2004: pp 198-207), and the authors concluded that this may be due to food reactions and food sensitivities occurring via a number of different mechanisms. For example, not all osteoarthritis patients show positive response to food A, and not all osteoarthritis patients show negative response to food B. Thus, even if an osteoarthritis patient shows positive response to food A, removal of food A from the patient's diet may not relieve the patient's osteoarthritis symptoms. In other words, it is not well determined whether food allergens used in the currently available tests are properly selected based on high probabilities of correlating sensitivities to those food allergens to osteoarthritis.

All publications identified herein are incorporated by reference to the same extent as if each individual publication or patent application were specifically and individually indicated to be incorporated by reference. Where a definition or use of a term in an incorporated reference is inconsistent or contrary to the definition of that term provided herein, the definition of that term provided herein applies and the definition of that term in the reference does not apply.

Thus, even though various tests for food sensitivities are known in the art, all or almost all of them suffer from one or more disadvantages. Therefore, there is still a need for improved compositions, devices, and methods of food sensitivity testing, especially for identification and possible elimination of foods that exacerbate or worsen symptoms for patients identified with or suspected of having osteoarthritis.

### Summary

The subject matter described herein provides systems and methods for testing food intolerance in patients diagnosed with or suspected to have osteoarthritis. One aspect of the disclosure is a test kit identifying food intolerances in patients diagnosed with or suspected to have osteoarthritis. The test kit includes a plurality of distinct food preparations coupled to individually addressable respective solid carriers. The plurality of distinct food preparations have an average discriminatory p-value of ≤ 0.07 as determined by raw p-value or an average discriminatory p-value of ≤ 0.10 as determined by FDR multiplicity adjusted p-value. In some embodiments, the average discriminatory p-value is determined from a patient test group that is not diagnosed or suspected of having osteoarthritis.

Another aspect of the embodiments described herein includes a method of testing food intolerance in patients diagnosed with or suspected to have osteoarthritis. The method includes a step of contacting a food preparation having at least one component with a bodily fluid of a patient that is diagnosed with or suspected to have osteoarthritis. The bodily fluid comprises an immunoglobulin (e.g., IgG, IgM, IgA, IgE) and is associated with gender identification. In one embodiment, the step of contacting is performed under conditions that allow immunoglobulin from the bodily fluid to bind to at least one component of the food preparation. The method continues with a step of measuring immunoglobulin bound to at least one component of the food preparation to obtain a signal, and then comparing the signal to a gender-stratified reference value for the food preparation using the gender identification to obtain a result. Then, the method also includes a step of updating or generating a report using the result.

Another aspect of the embodiments described herein includes a method of generating a test for food intolerance in patients diagnosed with or suspected to have osteoarthritis. The method includes a step of obtaining test results for a plurality of distinct food preparations. The test results are based on bodily fluids of patients diagnosed with or suspected to have osteoarthritis and bodily fluids of a control group not diagnosed with or not suspected to have osteoarthritis. The method also includes a step of stratifying the test results by gender for each of the distinct food preparations. Then the method continues with a step of assigning for a predetermined percentile rank a different cutoff value for male and female patients for each of the distinct food preparations.

Still another aspect of the embodiments described herein includes a use of a plurality of distinct food preparations coupled to individually addressable respective solid carriers in a diagnosis of osteoarthritis. The plurality of distinct food preparations are selected based on their average discriminatory p-value of ≤ 0.07 as determined by raw p-value or an average discriminatory p-value of ≤ 0.10 as determined by FDR multiplicity adjusted p-value.

Various objects, features, aspects and advantages of the embodiments described herein will become more apparent from the following detailed description of various embodiments, along with the accompanying figures in which like numerals represent like components.

### Brief Description of The Tables and Figures

**Table 1** shows a list of food items from which food preparations can be prepared.
**Table 2** shows statistical data of foods ranked according to 2-tailed FDR multiplicity-adjusted p-values.
**Table 3** shows statistical data of ELISA score by food and gender.
**Table 4** shows cutpoint values of foods for a predetermined percentile rank.
**Figure 1A** illustrates ELISA signal score of male osteoarthritis patients and control tested with chocolate.
**Figure 1B** illustrates a distribution of percentage of male osteoarthritis subjects exceeding the 90^{th} and 95^{th} percentile tested with chocolate.
**Figure 1C** illustrates a signal distribution in women along with the 95^{th} percentile cutoff as determined from the female control population tested with chocolate.
**Figure 1D** illustrates a distribution of percentage of female osteoarthritis subjects exceeding the 90^{th} and 95^{th} percentile tested with chocolate.
**Figure 2A** illustrates ELISA signal score of male osteoarthritis patients and control tested with grapefruit.
**Figure 2B** illustrates a distribution of percentage of male osteoarthritis subjects exceeding the 90^{th} and 95^{th} percentile tested with grapefruit.
**Figure 2C** illustrates a signal distribution in women along with the 95^{th} percentile cutoff as determined from the female control population tested with grapefruit.
**Figure 2D** illustrates a distribution of percentage of female osteoarthritis subjects exceeding the 90^{th} and 95^{th} percentile tested with grapefruit.
**Figure 3A** illustrates ELISA signal score of male osteoarthritis patients and control tested with honey.
**Figure 3B** illustrates a distribution of percentage of male osteoarthritis subjects exceeding the 90^{th} and 95^{th} percentile tested with honey.
**Figure 3C** illustrates a signal distribution in women along with the 95^{th} percentile cutoff as determined from the female control population tested with honey.
**Figure 3D** illustrates a distribution of percentage of female osteoarthritis subjects exceeding the 90^{th} and 95^{th} percentile tested with honey.
**Figure 4A** illustrates ELISA signal score of male osteoarthritis patients and control tested with malt.
**Figure 4B** illustrates a distribution of percentage of male osteoarthritis subjects exceeding the 90^{th} and 95^{th} percentile tested with malt.
**Figure 4C** illustrates a signal distribution in women along with the 95^{th} percentile cutoff as determined from the female control population tested with malt.
**Figure 4D** illustrates a distribution of percentage of female osteoarthritis subjects exceeding the 90^{th} and 95^{th} percentile tested with malt.
**Figures 5A** illustrates distributions of osteoarthritis subjects by number of foods that were identified as trigger foods at the 90^{th} percentile.
**Figures 5B** illustrates distributions of osteoarthritis subjects by number of foods that were identified as trigger foods at the 95^{th} percentile.
**Table 5A** shows raw data of osteoarthritis patients and control with number of positive results based on the 90^{th} percentile.
**Table 5B** shows raw data of osteoarthritis patients and control with number of positive results based on the 95^{th} percentile.
**Table 6A** shows statistical data summarizing the raw data of osteoarthritis patient populations shown in Table 5A.
**Table 6B** shows statistical data summarizing the raw data of osteoarthritis patient populations shown in Table 5B.
**Table 7A** shows statistical data summarizing the raw data of control populations shown in Table 5A.
**Table 7B** shows statistical data summarizing the raw data of control populations shown in Table 5B.
**Table 8A** shows statistical data summarizing the raw data of osteoarthritis patient populations shown in Table 5A transformed by logarithmic transformation.
**Table 8B** shows statistical data summarizing the raw data of osteoarthritis patient populations shown in Table 5B transformed by logarithmic transformation.
**Table 9A** shows statistical data summarizing the raw data of control populations shown in Table 5A transformed by logarithmic transformation.
**Table 9B** shows statistical data summarizing the raw data of control populations shown in Table 5B transformed by logarithmic transformation.
**Table 10A** shows statistical data of an independent T-test to compare the geometric mean number of positive foods between the osteoarthritis and non- osteoarthritis samples based on the 90^{th} percentile.
**Table 10B** shows statistical data of an independent T-test to compare the geometric mean number of positive foods between the osteoarthritis and non- osteoarthritis samples based on the 95^{th} percentile.
**Table 11A** shows statistical data of a Mann-Whitney test to compare the geometric mean number of positive foods between the osteoarthritis and non- osteoarthritis samples based on the 90^{th} percentile.
**Table 11B** shows statistical data of a Mann-Whitney test to compare the geometric mean number of positive foods between the osteoarthritis and non- osteoarthritis samples based on the 95^{th} percentile.

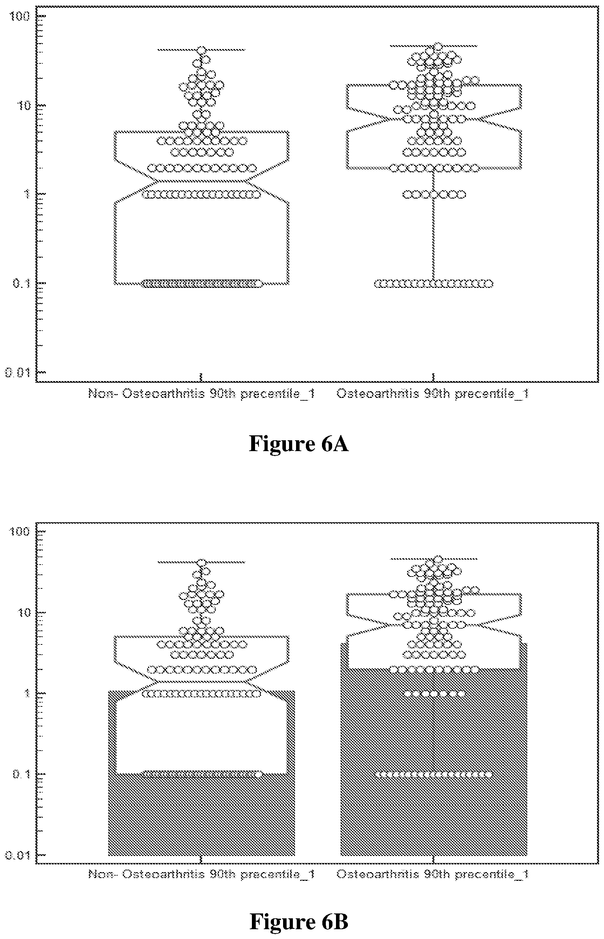

**Figure 6A** illustrates a box and whisker plot of data shown in Table 5A.
**Figure 6B** illustrates a notched box and whisker plot of data shown in Table 5A.

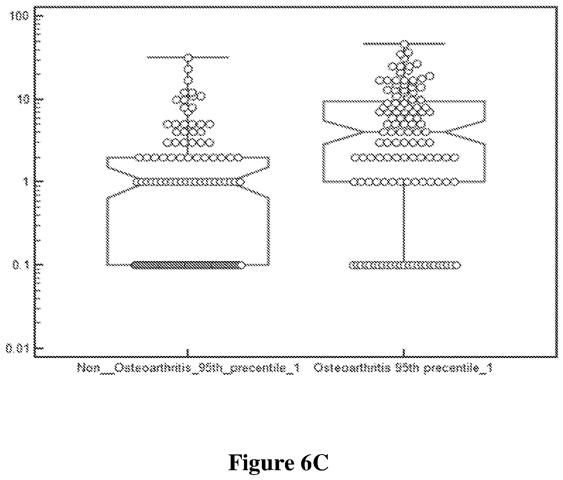

**Figure 6C** illustrates a box and whisker plot of data shown in Table 5B.

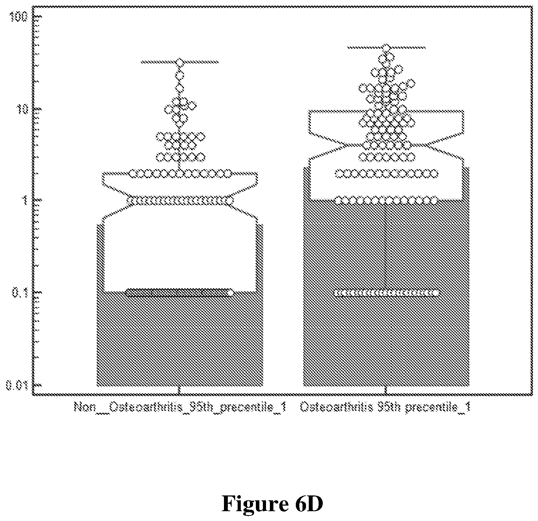

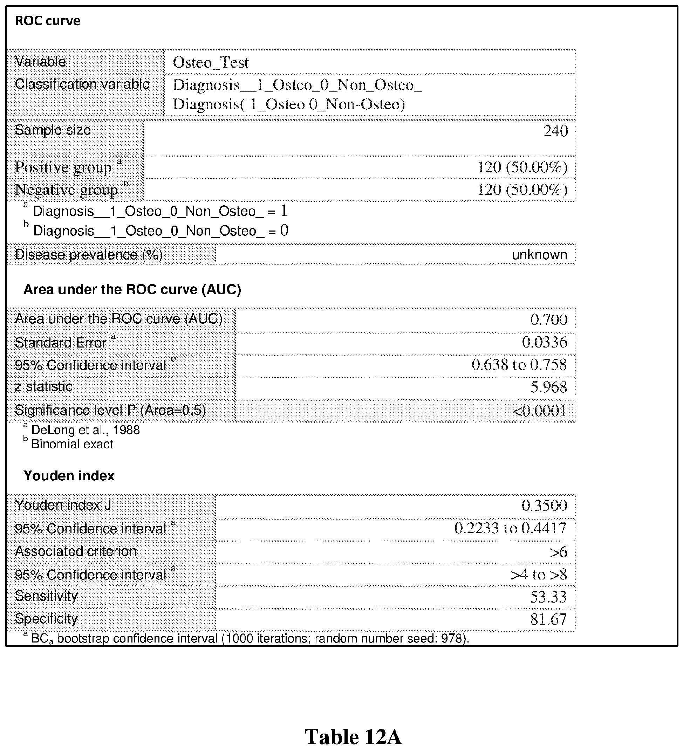

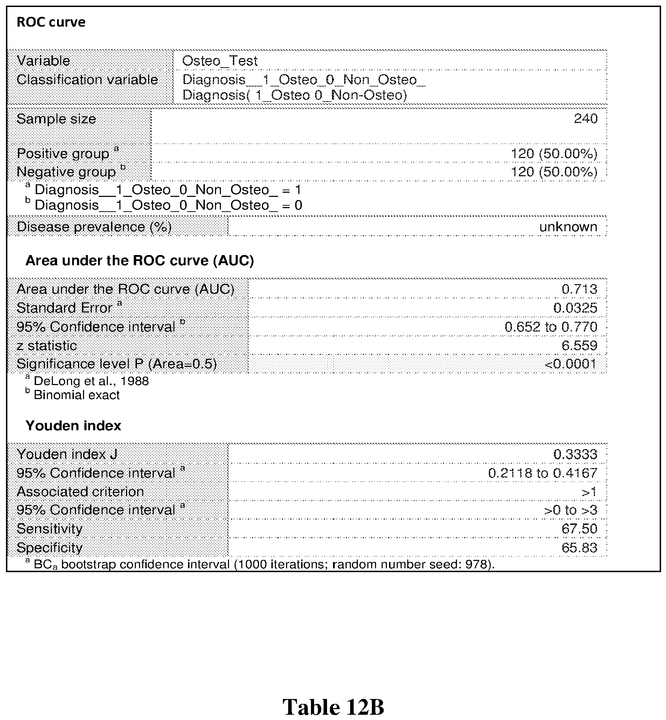

**Figure 6D** illustrates a notched box and whisker plot of data shown in Table 5B.
**Table 12A** shows statistical data of a Receiver Operating Characteristic (ROC) curve analysis of data shown in Tables 5A-11A.
**Table 12B** shows statistical data of a Receiver Operating Characteristic (ROC) curve analysis of data shown in Tables 5B-11B.

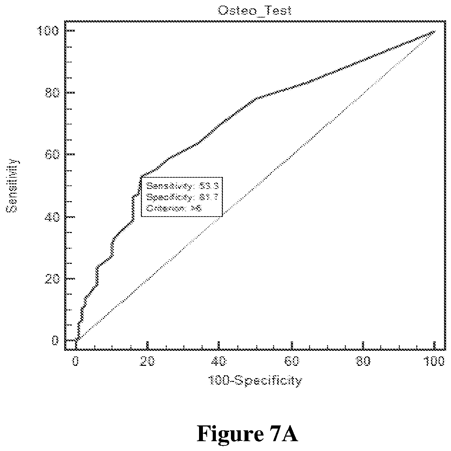

**Figure 7A** illustrates the ROC curve corresponding to the statistical data shown in Table 12B.

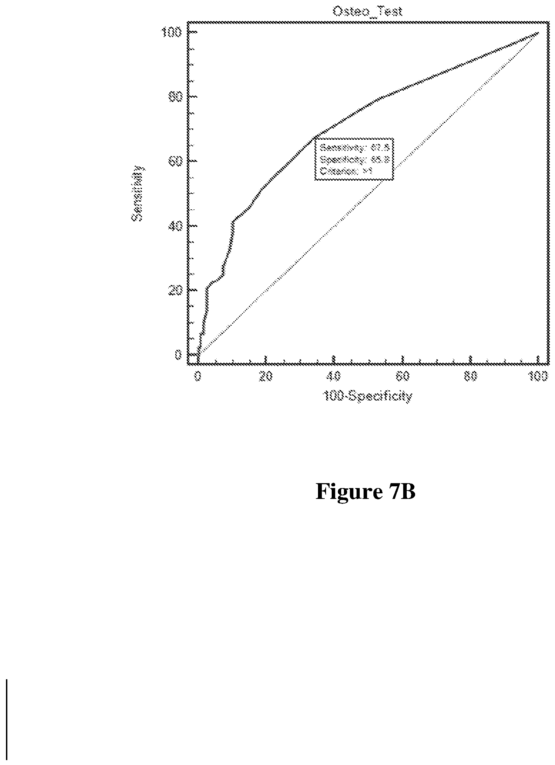

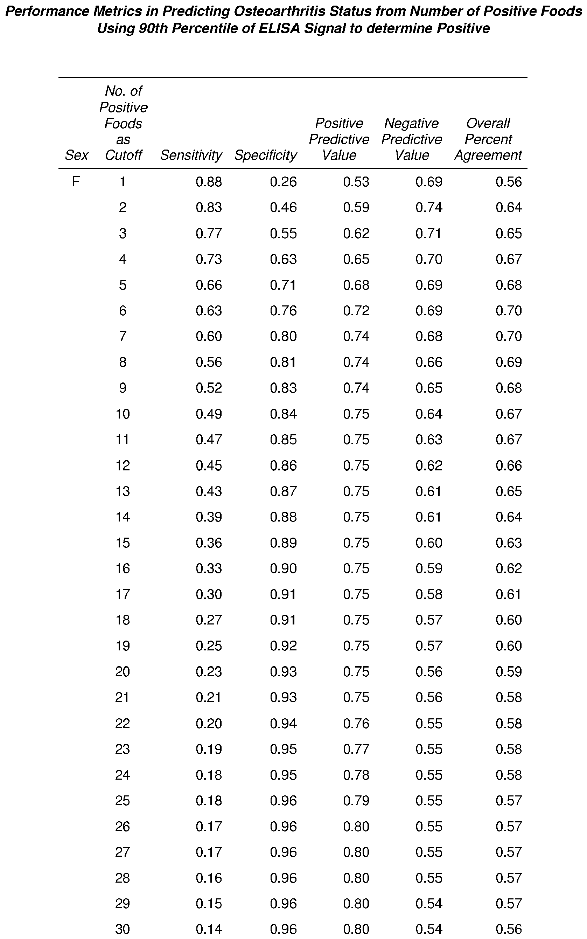

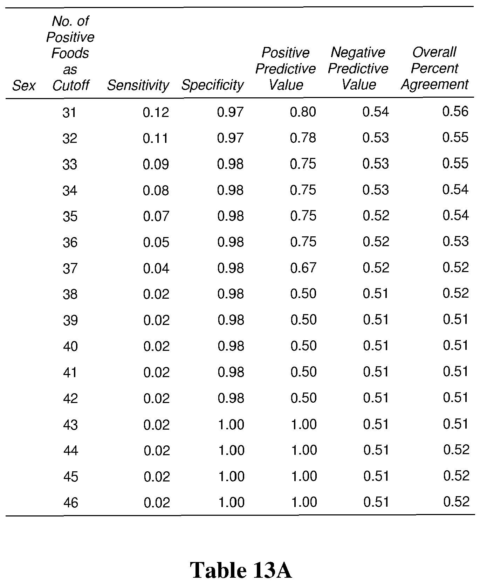

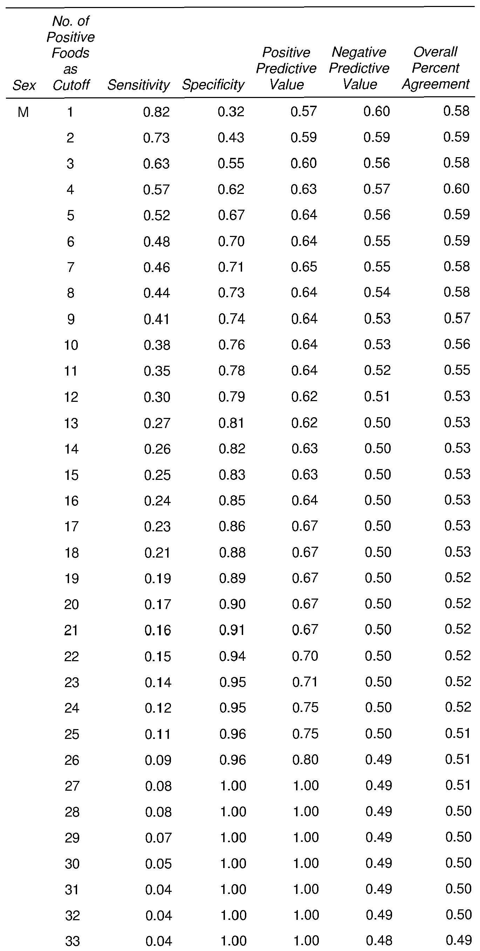

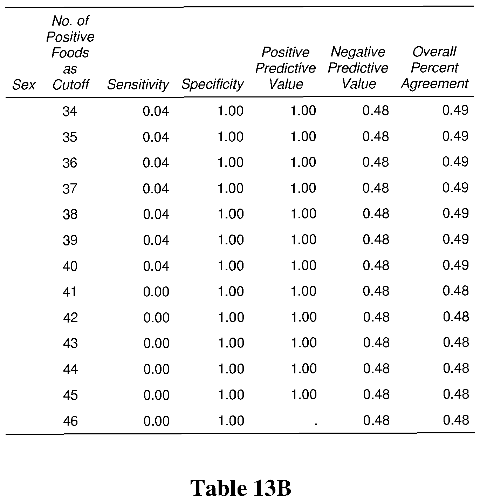

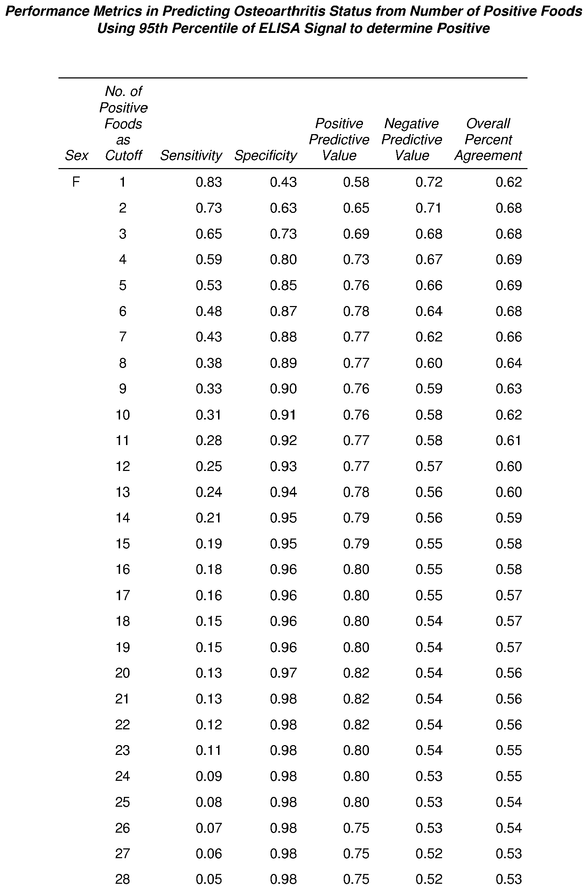

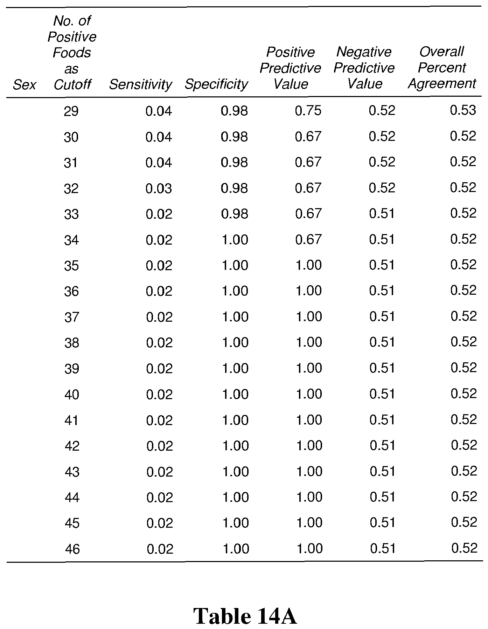

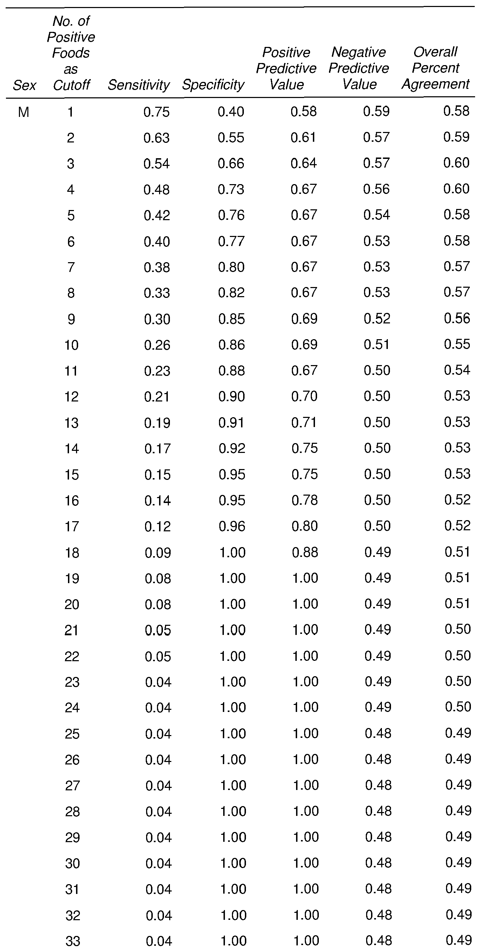

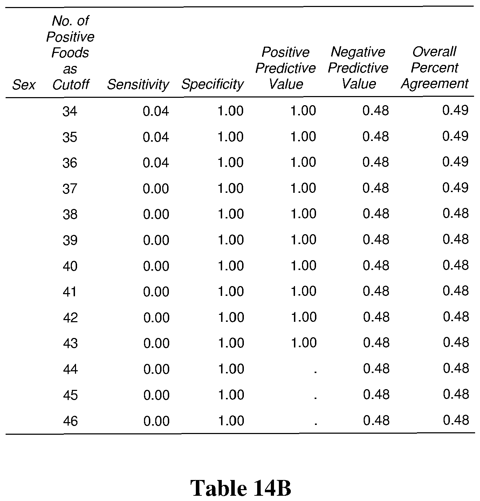

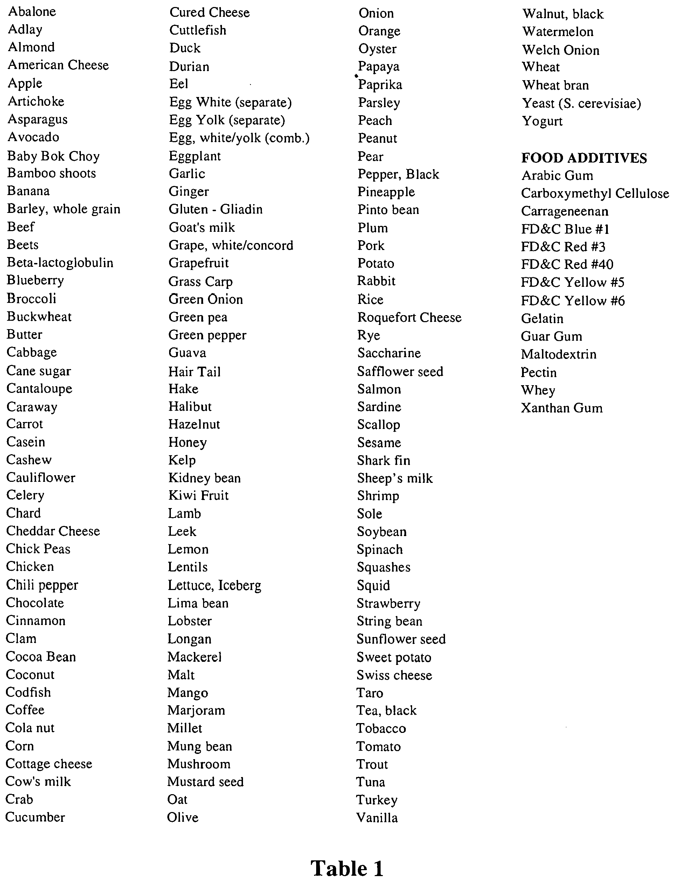

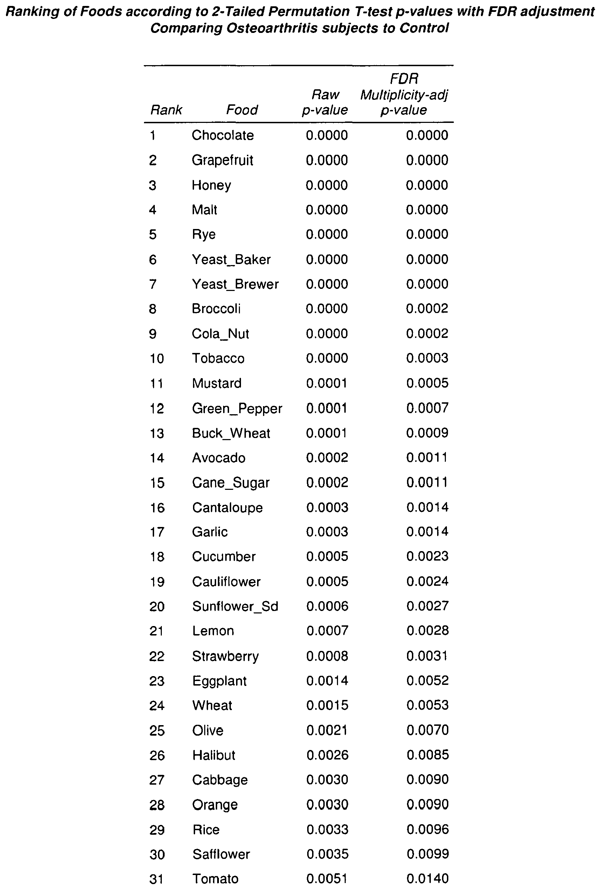

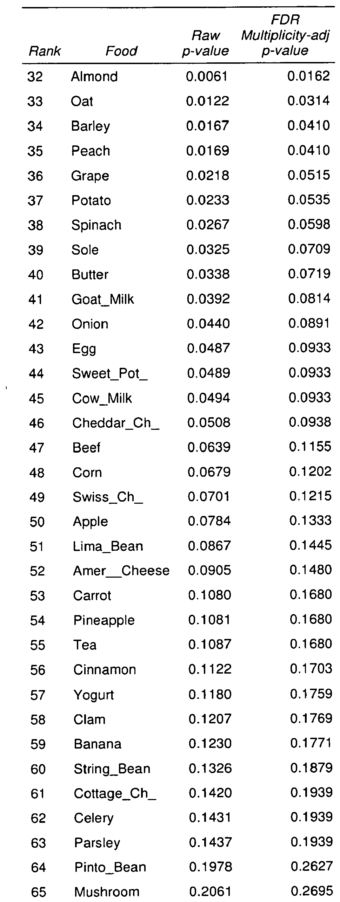

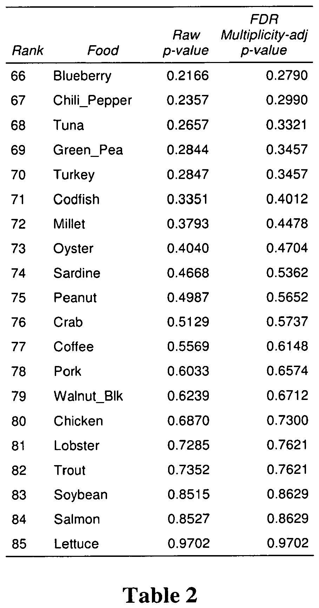

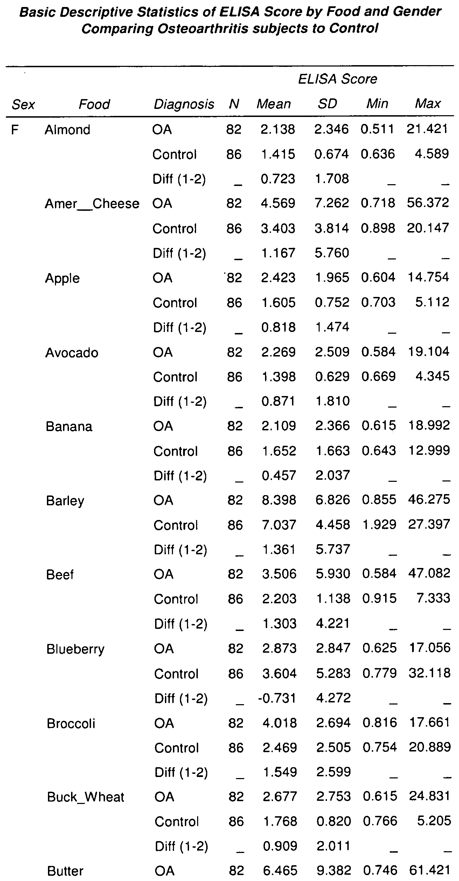

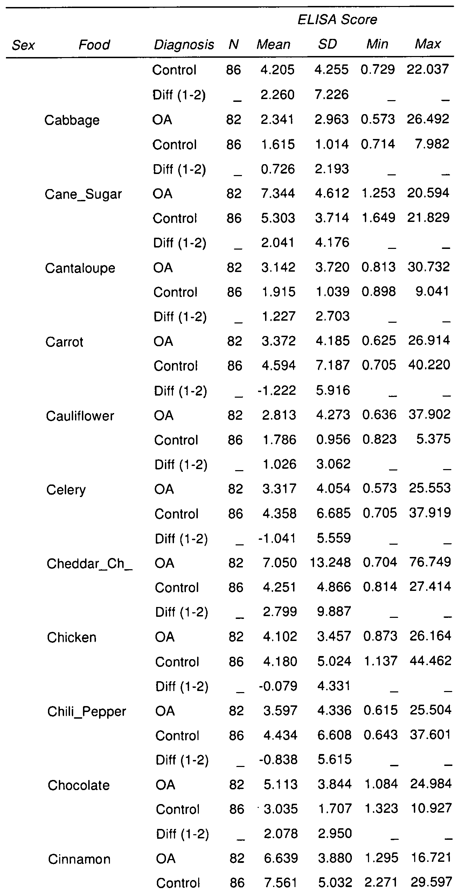

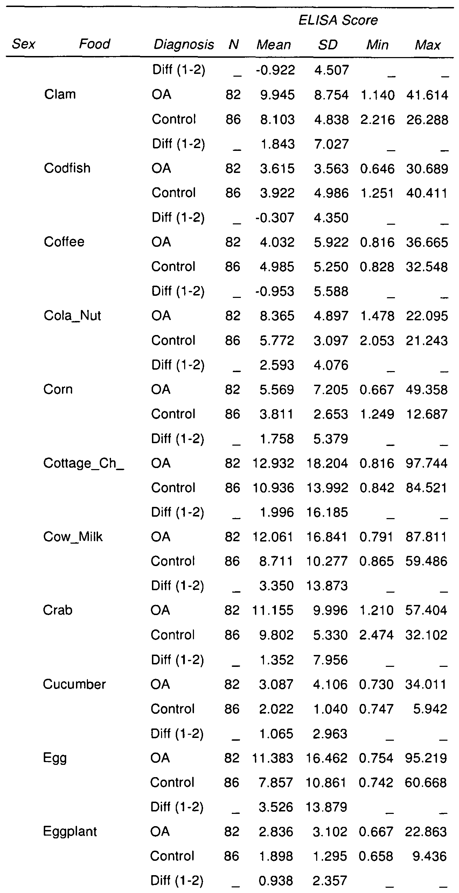

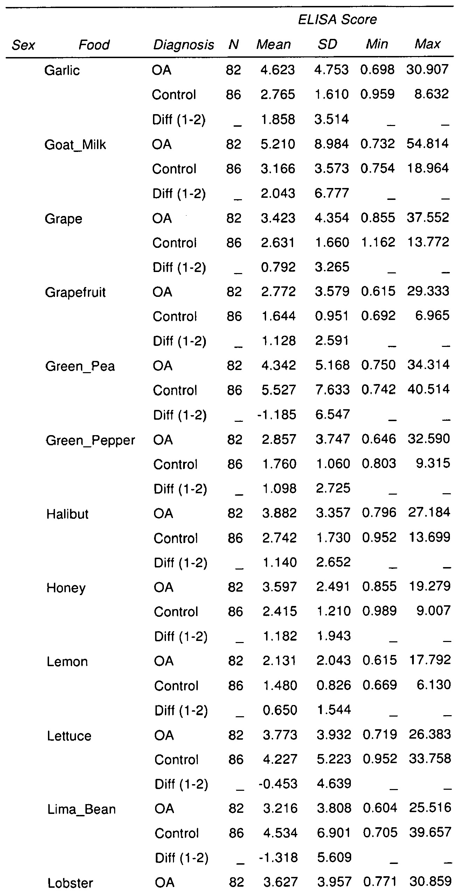

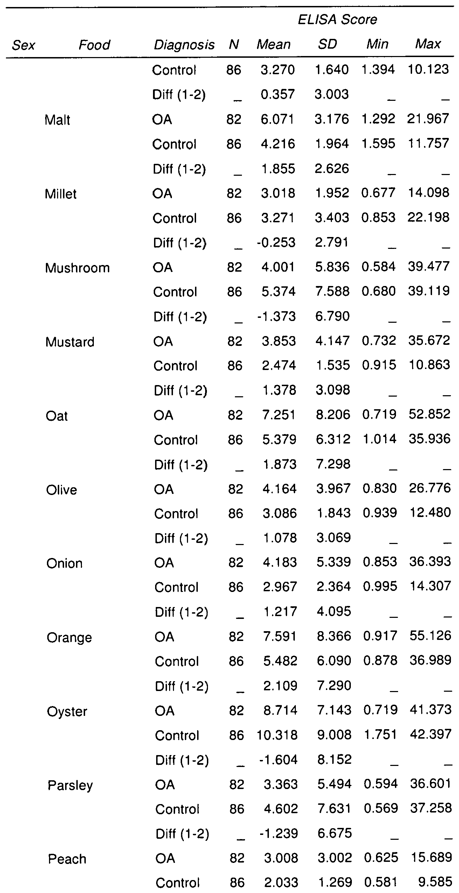

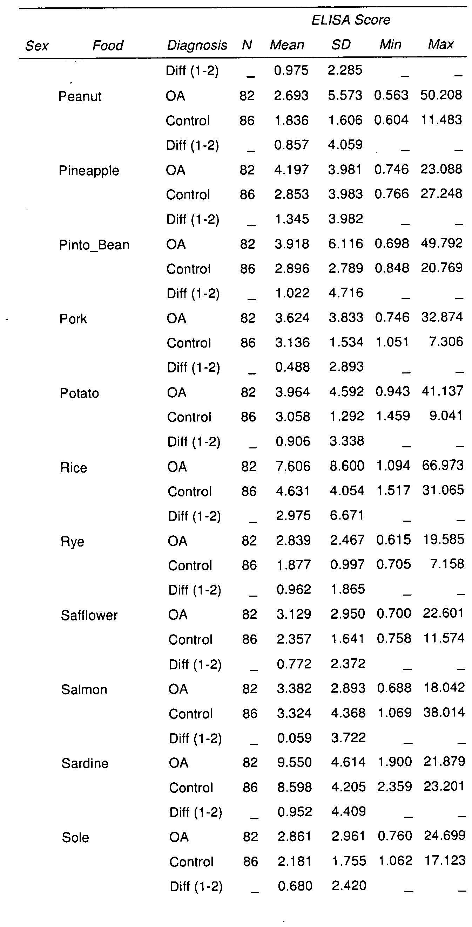

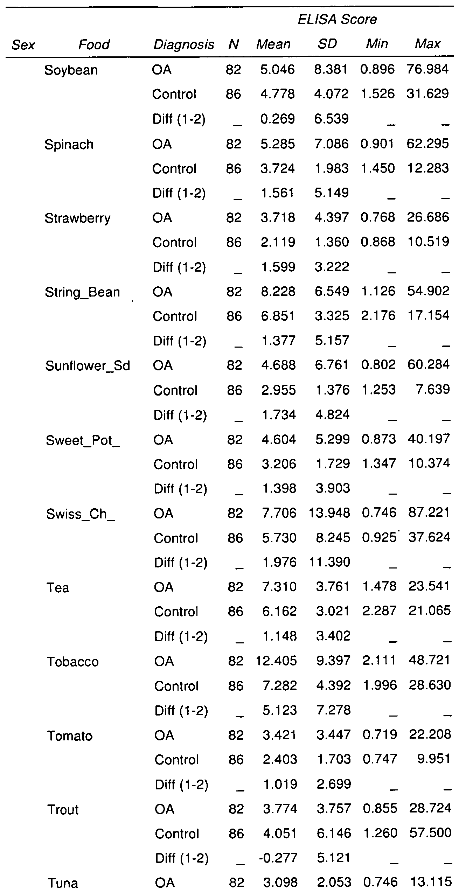

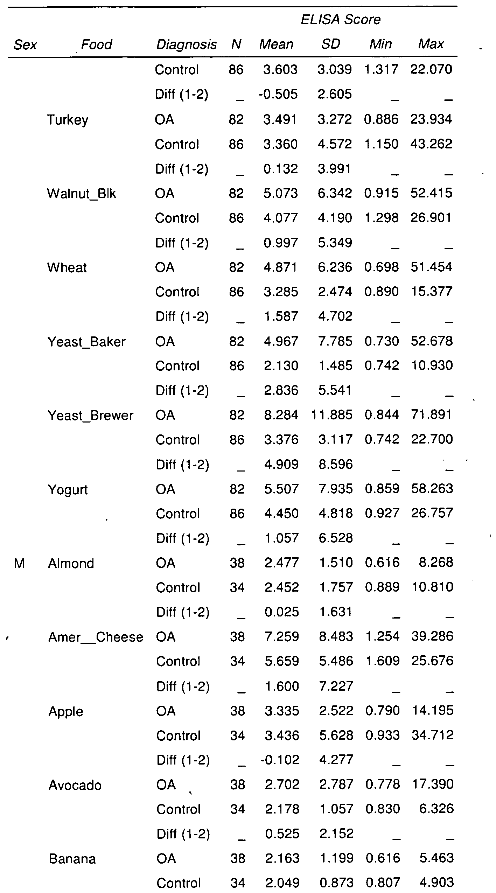

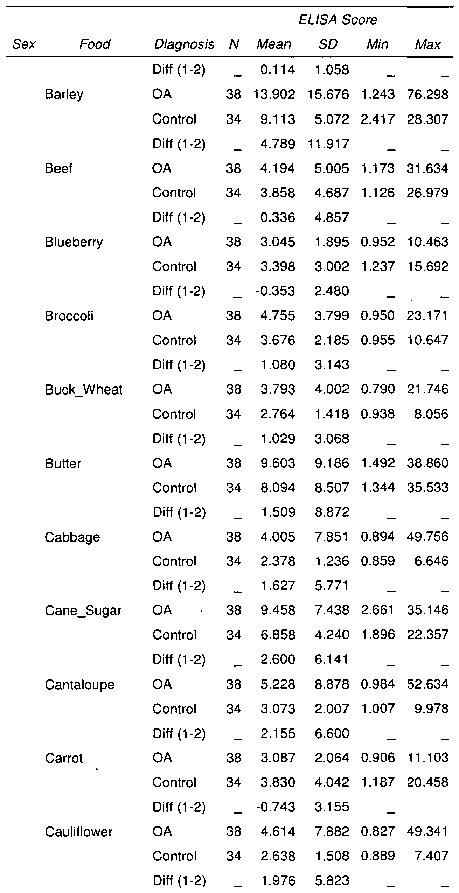

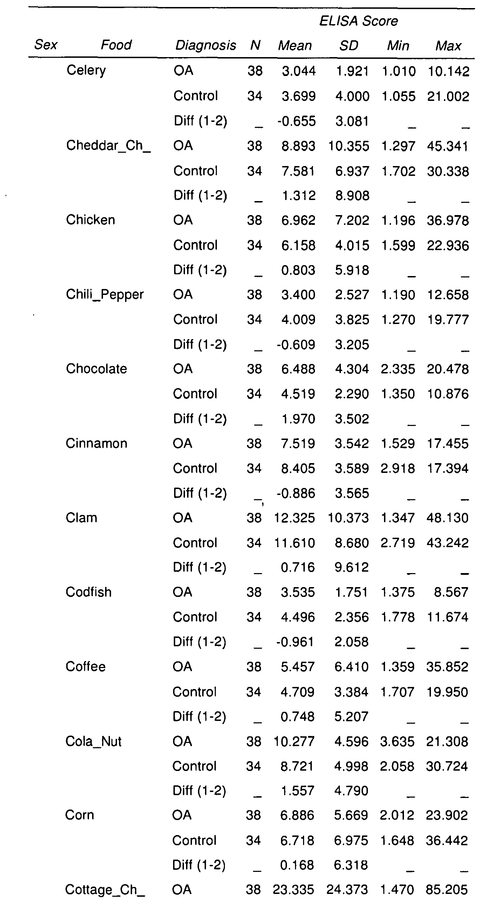

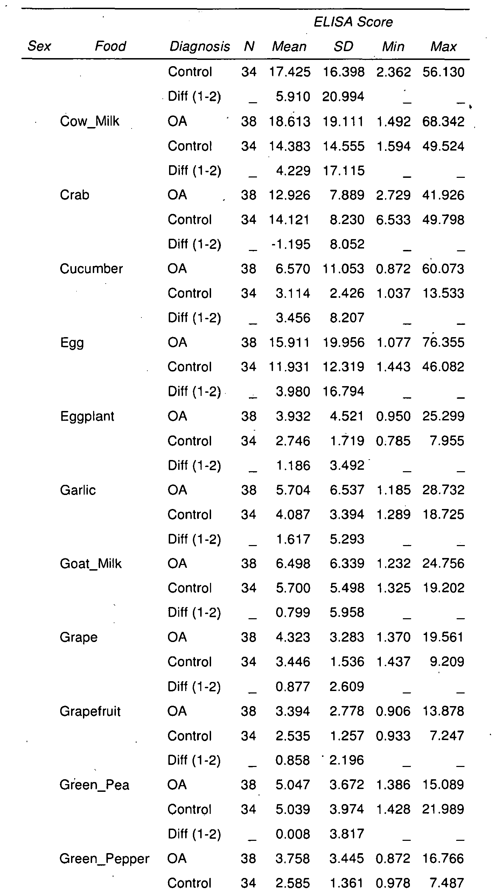

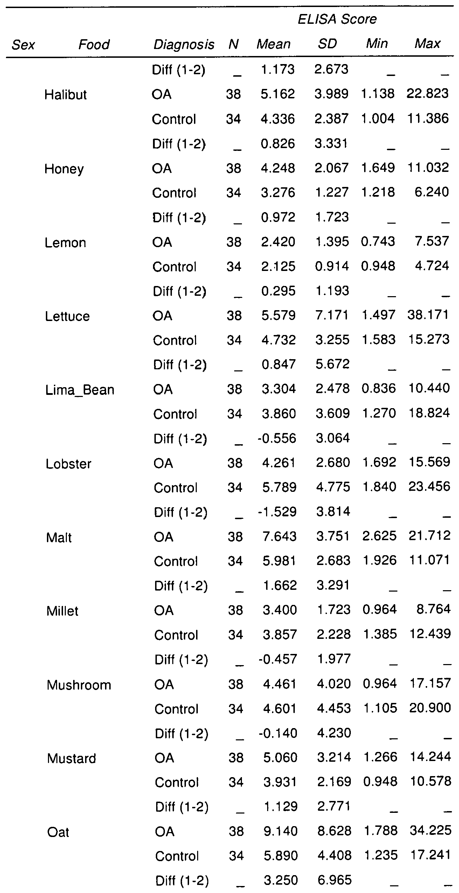

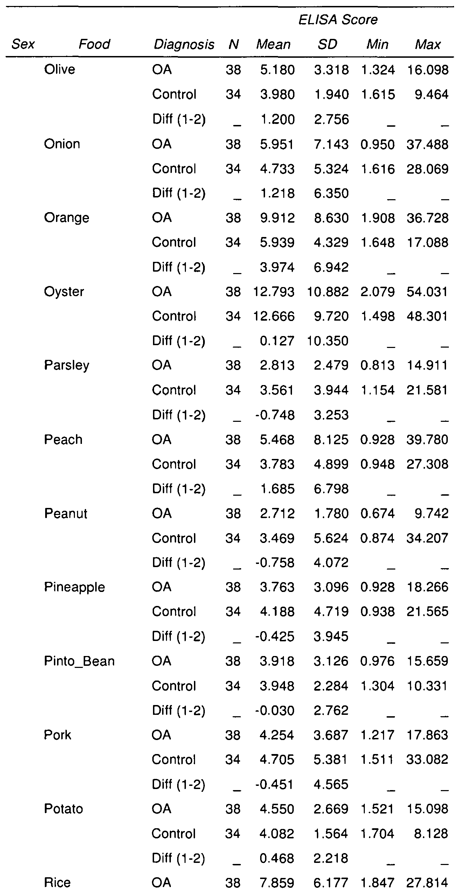

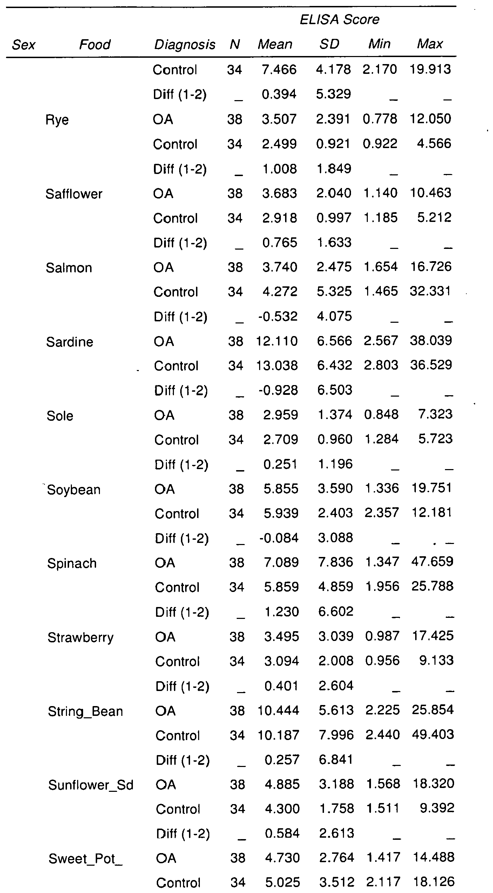

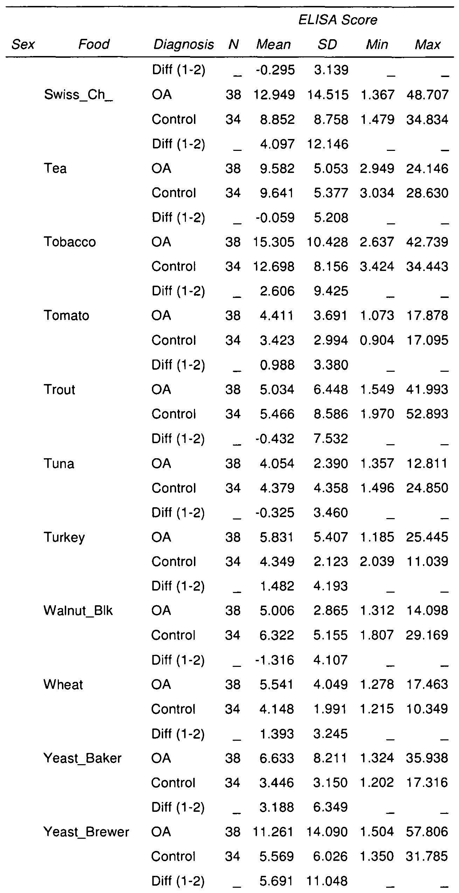

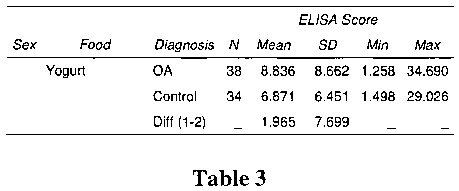

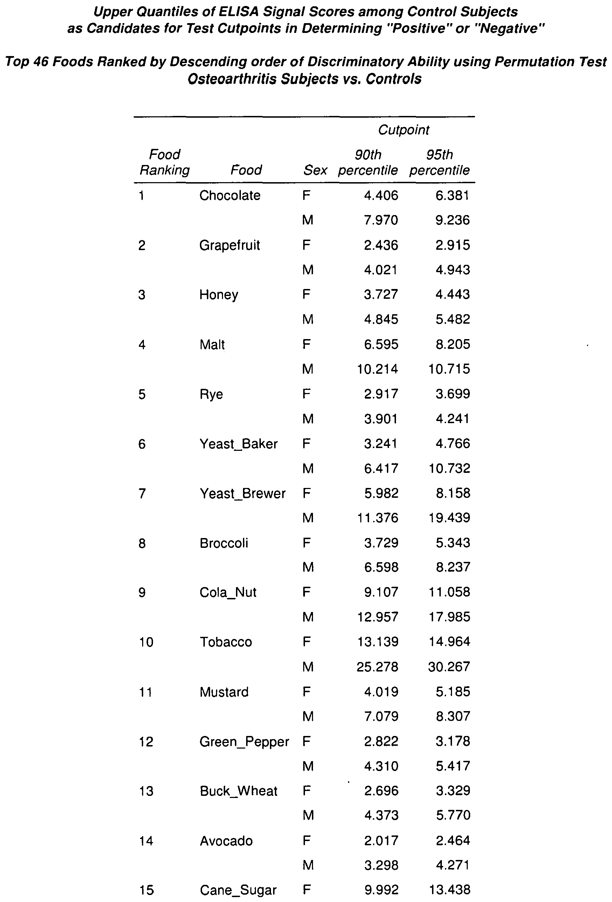

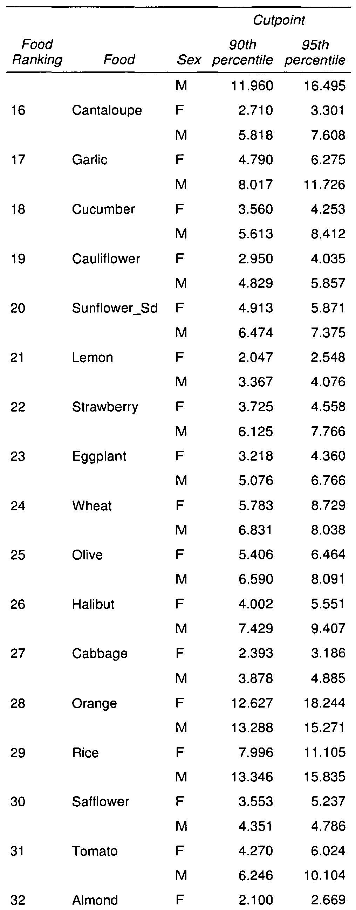

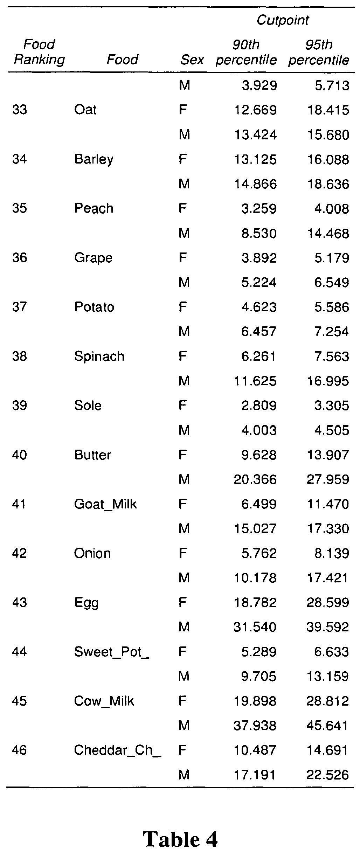

**Figure 7B** illustrates the ROC curve corresponding to the statistical data shown in Table 12B.
**Table 13A** shows a statistical data of performance metrics in predicting osteoarthritis status among female patients from number of positive foods based on the 90^{th} percentile.
**Table 13B** shows a statistical data of performance metrics in predicting osteoarthritis status among male patients from number of positive foods based on the 90^{th} percentile.
**Table 14A** shows a statistical data of performance metrics in predicting osteoarthritis status among female patients from number of positive foods based on the 95^{th} percentile.
**Table 14B** shows a statistical data of performance metrics in predicting osteoarthritis status among male patients from number of positive foods based on the 95^{th} percentile

### Detailed Description

The inventors have discovered that food preparations used in food tests to identify trigger foods in patients diagnosed with or suspected to have osteoarthritis are not equally well predictive and/or associated with osteoarthritis/osteoarthritis symptoms. Indeed, various experiments have revealed that among a wide variety of food items certain food items are highly predictive/associated with osteoarthritis whereas others have no statistically significant association with osteoarthritis. As used herein, the terms "trigger food" or "triggering food" refer to a food that is associated with, but not necessarily causative of signs and/or symptoms of osteoarthritis, and that - when eliminated from the diet of a patient diagnosed with or suspected to have osteoarthritis - reduces or alleviates signs and/or symptoms of osteoarthritis.

Even more unexpectedly, the inventors discovered that in addition to the high variability of food items, gender variability with respect to response in a test plays a substantial role in the determination of association or a food item with osteoarthritis. Consequently, based on the inventors' findings and further contemplations, test kits and methods are now presented with substantially higher predictive power in the choice of food items that could be eliminated for reduction of osteoarthritis signs and symptoms.

The following discussion provides many exemplary embodiments. Although each embodiment represents a single combination of certain elements, the concepts described herein considered to include all possible combinations of the disclosed elements. Thus if one embodiment comprises elements A, B, and C, and a second embodiment comprises elements B and D, then the embodiments described herein are also considered to include other remaining combinations of A, B, C, or D, even if not explicitly disclosed.

In some embodiments, the numbers expressing quantities or ranges, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the disclosure are approximations, the numerical values set forth in the specific examples are reported as precisely as practicable. The numerical values presented in some embodiments of the disclosure may contain certain errors resulting from the standard deviation found in their respective testing measurements. Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the disclosure and does not pose a limitation on the scope of what is otherwise claimed. No language in the specification should be construed as indicating any non-claimed element essential to the practice what is described herein.

Groupings of alternative elements or embodiments of the disclosure disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

In one aspect, the inventors therefore contemplate a test kit or test panel that is suitable for testing food intolerance in patients where the patient is diagnosed with or suspected to have osteoarthritis. It is contemplated that such test kit or panel will include a plurality of distinct food preparations (e.g., raw or processed extract, aqueous extract with optional co-solvent, which may or may not be filtered, etc.) that are coupled to individually addressable respective solid carriers *(e.g.,* in a form of an array or a micro well plate), wherein the distinct food preparations have an average discriminatory p-value of ≤ 0.07 as determined by raw p-value or an average discriminatory p-value of ≤ 0.10 as determined by FDR multiplicity adjusted p-value. As used herein, processed extracts includes food extracts made of food items that are mechanically or chemically modified (e.g., minced, heated, boiled, fermented, smoked, etc.).

In some embodiments, the numbers expressing quantities of ingredients, properties such as concentration, reaction conditions, and so forth, used to describe and claim certain embodiments of the disclosure are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the disclosure are approximations, the numerical values set forth in the specific examples are reported as precisely as practicable. The numerical values presented in some embodiments of the disclosure may contain certain errors resulting from the standard deviation found in their respective testing measurements. Moreover, and unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

While not limiting to the embodiments described herein, food preparations will typically be drawn from foods generally known or suspected to trigger signs or symptoms of osteoarthritis. Particularly suitable food preparations may be identified by the experimental procedures outlined below. Thus, it should be appreciated that the food items need not be limited to the items described herein, but that all items are contemplated that can be identified by the methods presented herein. Therefore, exemplary food preparations include at least one, at least two, at least four, at least eight, or at least 12 food preparations prepared from chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive. Additionally contemplated food preparations are prepared from halibut, cabbage, orange, rice (e.g., brown rice, white rice, etc.), safflower, tomato, almond, oat, barley, peach, grape, potato, spinach, sole, and butter. Still further especially contemplated food items and food additives from which food preparations can be prepared are listed in **Table 1.**

Using bodily fluids from patients diagnosed with or suspected to have osteoarthritis and healthy control group individuals (i.e., those not diagnosed with or not suspected to have osteoarthritis), numerous additional food items may be identified. Such identified food items will have high discriminatory power and as such have a p-value of ≤ 0.15, or of ≤ 0.10, or of ≤ 0.05 as determined by raw p-value, and/or a p-value of ≤ 0.10, or of ≤ 0.08, and or of ≤ 0.07 as determined by False Discovery Rate (FDR) multiplicity adjusted p-value.

Therefore, where a panel has multiple food preparations, it is contemplated that the plurality of distinct food preparations has an average discriminatory p-value of ≤ 0.05 as determined by raw p-value or an average discriminatory p-value of ≤ 0.08 as determined by FDR multiplicity adjusted p-value, or an average discriminatory p-value of ≤ 0.025 as determined by raw p-value or an average discriminatory p-value of ≤ 0.07 as determined by FDR multiplicity adjusted p-value. In one aspect, it should be appreciated that the FDR multiplicity adjusted p-value may be adjusted for at least one of age and gender, and sometimes adjusted for both age and gender. On the other hand, where a test kit or panel is stratified for use with a single gender, it is also contemplated that in a test kit or panel at least 50% (and more typically 70% or all) of the plurality of distinct food preparations, when adjusted for a single gender, have an average discriminatory p-value of ≤ 0.07 as determined by raw p-value or an average discriminatory p-value of ≤ 0.10 as determined by FDR multiplicity adjusted p-value. Furthermore, it should be appreciated that other stratifications (e.g., dietary preference, ethnicity, place of residence, genetic predisposition or family history, etc.) are also contemplated, and the PHOSITA will be readily appraised of the appropriate choice of stratification.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the disclosure and does not pose a limitation on the scope of the claims. No language in the specification should be construed as indicating any non-claimed element essential to the practice of the embodiments described herein.

Of course, it should be noted that the particular format of the test kit or panel may vary considerably and contemplated formats include micro well plates, a microfluidic device, dip sticks, membrane-bound arrays, etc. Consequently, the solid carrier to which the food preparations are coupled may include wells of a multiwall plate, a microfluidic device, a (*e.g.,* color-coded or magnetic) bead, or an adsorptive film (e.g., nitrocellulose or micro/nanoporous polymeric film), a chemical sensor, or an electrical sensor, (e.g., a printed copper sensor or microchip). In some embodiments, it is also contemplated that a suitable solid carrier for molecular absorption and signal detection by a light detector (e.g., surface plasmon resonance, etc.) can be used.

Consequently, the inventors also contemplate a method of testing food intolerance in patients that are diagnosed with or suspected to have osteoarthritis. Most typically, such methods will include a step of contacting a food preparation with a bodily fluid (e.g., whole blood, plasma, serum, saliva, or a fecal suspension) of a patient that is diagnosed with or suspected to have osteoarthritis, and wherein the bodily fluid is associated with a gender . identification. As noted before, the step of contacting is performed under conditions that allow immunoglobulin (IgG or IgE or IgA or IgM, or combinations of any of those) from the bodily fluid to bind to at least one component of the food preparation, and the immunoglobulin bound to the component(s) of the food preparation are then quantified/measured to obtain a signal. In one embodiment, the signal is then compared against a gender-stratified reference value (e.g., at least a 90th percentile value) for the food preparation using the gender identification to obtain a result, which is then used to update or generate a report. In one embodiment, the report can be generated as an aggregate result of individual assay results.

Most commonly, such methods will not be limited to a single food preparation, but will employ multiple different food preparations. As noted before, suitable food preparations can be identified using various methods as described below, however, one exemplary group of food preparations include chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive. Additionally contemplated food preparations are prepared from halibut, cabbage, orange, rice (*e.g.*, brown rice, white rice, etc.), safflower, tomato, almond, oat, barley, peach, grape, potato, spinach, sole, and butter. Still further especially contemplated food items and food additives from which food preparations can be prepared are listed in **Table 1.** As also noted above, it is contemplated that at least some, or all of the different food preparations have an average discriminatory p-value of ≤ 0.07 (or ≤ 0.05, or ≤ 0.025) as determined by raw p-value, and/or or an average discriminatory p-value of ≤ 0.10 (or ≤ 0.08, or ≤ 0.07) as determined by FDR multiplicity adjusted p-value.

While it is contemplated that food preparations are prepared from a single food items as crude extracts, or crude filtered extracts, it is contemplated that food preparations can be prepared from mixtures of a plurality of food items (*e.g.,* a mixture of citrus comprising lemon, orange, and a grapefruit, a mixture of yeast comprising baker's yeast and brewer's yeast, a mixture of rice comprising a brown rice and white rice, a mixture of sugars comprising honey, malt, and cane sugar. In some embodiments, it is also contemplated that food preparations can be prepared from purified food antigens or recombinant food antigens.

While it is contemplated that food preparation is immobilized on a solid surface (typically in an addressable manner), the step of measuring the IgG or other type of antibody bound to the component of the food preparation can be also performed via an immunoassay test *(e.g.,* ELISA test, antibody capture enzyme immunoassay, other types of antibody capture assays, etc.)

Viewed from a different perspective, the inventors also contemplate a method of generating a test for food intolerance in patients diagnosed with or suspected to have osteoarthritis. Because the test is applied to patients already diagnosed with or suspected to have osteoarthritis, the authors do not contemplate that the method has a primary diagnostic purpose for osteoarthritis. Instead, the method is for identifying triggering food items among already diagnosed or suspected osteoarthritis patients. Such test will typically include a step of obtaining one or more test results *(e.g.,* ELISA, antibody capture enzyme immunoassay) for various distinct food preparations, wherein the test results are based on bodily fluids *(e.g.,* blood saliva, fecal suspension) of patients diagnosed with or suspected to have osteoarthritis and bodily fluids of a control group not diagnosed with or not suspected to have osteoarthritis. In one embodiment, the test results are then stratified by gender for each of the distinct food preparations, a different cutoff value for male and female patients for each of the distinct food preparations *(e.g.,* cutoff value for male and female patients has a difference of at least 10% (abs)) is assigned for a predetermined percentile rank *(e.g.,* 90th or 95th percentile).

As noted earlier, and while not limiting to the embodiments described herein, it is contemplated that the distinct food preparations include at least two (or six, or ten, or 15) food preparations prepared from food items selected from the group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive. Additionally contemplated food preparations are prepared from halibut, cabbage, orange, rice *(e.g.,* brown rice, white rice, etc.), safflower, tomato, almond, oat, barley, peach, grape, potato, spinach, sole, and butter. Still further especially contemplated food items and food additives from which food preparations can be prepared are listed in **Table 1.** On the other hand, where new food items are tested, it should be appreciated that the distinct food preparations include a food preparation prepared from items other than chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive. Regardless of the particular choice of food items, it is generally contemplated that the distinct food preparations have an average discriminatory p-value of ≤ 0.07 (or ≤ 0.05, or ≤ 0.025) as determined by raw p-value or an average discriminatory p-value of ≤ 0.10 (or ≤ 0.08, or ≤ 0.07) as determined by FDR multiplicity adjusted p-value. Exemplary aspects and protocols, and considerations are provided in the experimental description below.

The invention also provides the following embodiments:
1. A test kit for testing food intolerance in patients diagnosed with or suspected to have osteoarthritis, comprising:
   a plurality of distinct food preparations coupled to individually addressable respective solid carriers; wherein the plurality of distinct food preparations has an average discriminatory p-value of ≤ 0.07 as determined by raw p-value or an average discriminatory p-value of ≤ 0.10 as determined by FDR multiplicity adjusted p-value, wherein the average discriminatory p-value is determined from a patient test group that is not diagnosed or suspected of having osteoarthritis.
2. The test kit of embodiment 1 wherein the plurality of food preparations includes at least two food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
3. The test kit of embodiment 1 wherein the plurality of food preparations includes at least four food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
4. The test kit of embodiment 1 wherein the plurality of food preparations includes at least eight food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
5. The test kit of embodiment 1 wherein the plurality of food preparations includes at least 12 food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
6. The test kit of embodiment 1 wherein the plurality of distinct food preparations has an average discriminatory p-value of ≤ 0.05 as determined by raw p-value or an average discriminatory p-value of ≤ 0.08 as determined by FDR multiplicity adjusted p-value.
7. The test kit of any one of embodiments 1-5 wherein the plurality of distinct food preparations has an average discriminatory p-value of ≤ 0.05 as determined by raw p-value or an average discriminatory p-value of ≤ 0.08 as determined by FDR multiplicity adjusted p-value.
8. The test kit of embodiment 1 wherein the plurality of distinct food preparations has an average discriminatory p-value of ≤ 0.025 as determined by raw p-value or an average discriminatory p-value of ≤ 0.07 as determined by FDR multiplicity adjusted p-value.
9. The test kit of any one of embodiments 1-5 wherein the plurality of distinct food preparations has an average discriminatory p-value of ≤ 0.025 as determined by raw p-value or an average discriminatory p-value of ≤ 0.07 as determined by FDR multiplicity adjusted p-value.
10. The test kit of embodiment 1 wherein FDR multiplicity adjusted p-value is adjusted for at least one of age and gender.
11. The test kit of any one of embodiments 1-8 wherein FDR multiplicity adjusted p-value is adjusted for at least one of age and gender.
12. The test kit of embodiment 1 wherein FDR multiplicity adjusted p-value is adjusted for age and gender.
13. The test kit of any one of embodiments 1-8 wherein FDR multiplicity adjusted p-value is adjusted for age and gender.
14. The test kit of embodiment 1 wherein at least 50% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value.
15. The test kit of any one of embodiments 1-13 wherein at least 50% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value.
16. The test kit of embodiment 1 wherein at least 70% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value.
17. The test kit of any one of the embodiments 1-13 wherein at least 70% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value.
18. The test kit of embodiment 1 wherein all of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value.
19. The test kit of any one of the embodiments 1-17 wherein all of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value.
20. The test kit of embodiment 1 wherein the plurality of distinct food preparations is crude aqueous extracts.
21. The test kit of any one of the embodiments 1-19 wherein the plurality of distinct food preparations is crude aqueous extracts.
22. The test kit of embodiment 1 wherein the plurality of distinct food preparations is processed aqueous extracts.
23. The test kit of any one of the embodiments 1-21 wherein the plurality of distinct food preparations is processed aqueous extracts.
24. The test kit of embodiment 1 wherein the solid carrier is a well of a multiwall plate, a bead, an electrical sensor, a chemical sensor, a microchip or an adsorptive film.
25. The test kit of any one of the embodiments 1-23 wherein the solid carrier is a well of a multiwall plate, a bead, an electrical sensor, a chemical sensor, a microchip or an adsorptive film.
26. A method of testing food intolerance in patients diagnosed with or suspected to have osteoarthritis, comprising:
   contacting a food preparation having at least one component with a bodily fluid of a patient that is diagnosed with or suspected to have osteoarthritis, wherein the bodily fluid comprises immunoglobulins, wherein the bodily fluid is associated with a gender identification, and wherein the contacting is performed under conditions that allow at least a portion of the immunoglobulins to bind to the at least one component;
   measuring the portion of the immunoglobulins bound to the at least one component of the food preparation to obtain a signal;
   comparing the signal to a gender-stratified reference value for the food preparation using the gender identification to obtain a result; and
   updating or generating a report using the result.
27. The method of embodiment 26, wherein the bodily fluid of the patient is whole blood, plasma, serum, saliva, or a fecal suspension.
28. The method of embodiment 26, wherein the contacting a food preparation is performed with a multiplexed assay comprised of plurality of distinct food preparations.
29. The method of embodiment 26 or embodiment 27 wherein contacting a food preparation is performed with a multiplexed assay comprised of plurality of distinct food preparations.
30. The method of embodiment 28 wherein the plurality of distinct food preparations is prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
31. The method of any of the embodiments 28-29 wherein the plurality of distinct food preparations is prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
32. The method of embodiment 28 wherein the plurality of distinct food preparations has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
33. The method of any of the embodiments 28-29 wherein the plurality of distinct food preparations has an average discriminatory p-value of ≤ 0.07 as determined by raw p-value or an average discriminatory p-value of ≤ 0.10 as determined by FDR multiplicity adjusted p-value, and
   wherein the average discriminatory p-value is determined from a patient test group that is not diagnosed or suspected of having osteoarthritis.
34. The method of embodiment 28 wherein the plurality of distinct food preparations has an average discriminatory p-value of ≤ 0.05 as determined by raw p-value or an average discriminatory p-value of ≤ 0.08 as determined by FDR multiplicity adjusted p-value, and wherein the average discriminatory p-value is determined from a patient test group that is not diagnosed or suspected of having osteoarthritis.
35. The method of any of the embodiments 28-29 wherein the plurality of distinct food preparations has an average discriminatory p-value of ≤ 0.05 as determined by raw p-value or an average discriminatory p-value of ≤ 0.08 as determined by FDR multiplicity adjusted p-value, and wherein the average discriminatory p-value is determined from a patient test group that is not diagnosed or suspected of having osteoarthritis.
36. The method of embodiment 28 wherein the plurality of distinct food preparations has an average discriminatory p-value of ≤ 0.025 as determined by raw p-value or an average discriminatory p-value of ≤ 0.07 as determined by FDR multiplicity adjusted p-value, and wherein the average discriminatory p-value is determined from a patient test group that is not diagnosed or suspected of having osteoarthritis.
37. The method of any of the embodiments 28-29 wherein the plurality of distinct food preparations has an average discriminatory p-value of ≤ 0.025 as determined by raw p-value or an average discriminatory p-value of ≤ 0.07 as determined by FDR multiplicity adjusted p-value, and wherein the average discriminatory p-value is determined from a patient test group that is not diagnosed or suspected of having osteoarthritis.
38. The method of embodiment 28 wherein all of the plurality of distinct food preparations has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
39. The method of any of the embodiments 28-29 wherein all of the plurality of distinct food preparations has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
40. The method of embodiment 26 wherein the food preparation is immobilized on a solid surface, optionally in an addressable manner.
41. The method of any of the embodiments 26-39 wherein the food preparation is immobilized on a solid surface, optionally in an addressable manner.
42. The method of embodiment 26 wherein the measuring the portion of the immunoglobulins bound to the at least one component of the food preparation is performed via an immunoassay test.
43. The method of any of the embodiments 26-41 wherein the measuring the portion of the immunoglobulins bound to the at least one component of the food preparation is performed via immunoassay test.
44. The method of embodiment 26 wherein the gender-stratified reference value for the food preparation is an at least a 90th percentile value.
45. The method of any of the embodiments 26-43 wherein the gender-stratified reference value for the food preparation is an at least a 90th percentile value.
46. A method of generating a test for food intolerance in patients diagnosed with or suspected to have osteoarthritis, comprising:
   obtaining test results for a plurality of distinct food preparations, wherein the test results are derived from a process that includes contacting the plurality of distinct food preparations with bodily fluids from males and females of a patient group diagnosed with or suspected to have osteoarthritis, and bodily fluids from males and females of a control group not diagnosed with or not suspected to have osteoarthritis;
   stratifying the test results by gender for the plurality of distinct food preparations; and assigning for a predetermined percentile rank a different cutoff value for males of the patient group and females of the patient group, for the plurality of the distinct food preparations.
47. The method of embodiment 46 wherein the test result is an antibody-based result.
48. The method of embodiment 46 wherein the plurality of distinct food preparations includes at least two food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
49. The method of embodiment 46 or embodiment 47 wherein the plurality of distinct food preparations includes at least two food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
50. The method of embodiment 46 wherein the plurality of distinct food preparations includes at least six food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
51. The method of any of embodiment 46 or embodiment 47 wherein the plurality of distinct food preparations includes at least six food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
52. The method of embodiment 46 wherein the plurality of distinct food preparations includes a food preparation prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
53. The method of any of embodiment 46 or 47 wherein the plurality of distinct food preparations includes a food preparation prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
54. The method of embodiment 46 wherein the plurality of distinct food preparations has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
55. The method of any of embodiments 46-53 wherein the plurality of distinct food preparations has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
56. The method of embodiment 46 wherein the plurality of different food preparations has an average discriminatory p-value of ≤ 0.05 as determined by raw p-value or an average discriminatory p-value of ≤ 0.08 as determined by FDR multiplicity adjusted p-value, and wherein the average discriminatory p-value is determined from a patient test group that is not diagnosed or suspected of having osteoarthritis.
57. The method of any of embodiments 46-53 wherein the plurality of different food preparations has an average discriminatory p-value of ≤ 0.05 as determined by raw p-value or an average discriminatory p-value of ≤ 0.08 as determined by FDR multiplicity adjusted p-value, and wherein the average discriminatory p-value is determined from a patient test group that is not diagnosed or suspected of having osteoarthritis.
58. The method of embodiment 46 wherein the plurality of different food preparations has an average discriminatory p-value of ≤ 0.025 as determined by raw p-value or an average discriminatory p-value of ≤ 0.07 as determined by FDR multiplicity adjusted p-value, and wherein the average discriminatory p-value is determined from a patient test group that is not diagnosed or suspected of having osteoarthritis.
59. The method of any of embodiments 46-53 wherein the plurality of different food preparations has an average discriminatory p-value of ≤ 0.025 as determined by raw p-value or an average discriminatory p-value of ≤ 0.07 as determined by FDR multiplicity adjusted p-value, and wherein the average discriminatory p-value is determined from a patient test group that is not diagnosed or suspected of having osteoarthritis.
60. The method of embodiment 46 wherein the bodily fluid of the patient is whole blood, plasma, serum, saliva, or a fecal suspension.
61. The method of any of embodiments 46-59 wherein the bodily fluid of the patient is whole blood, plasma, serum, saliva, or a fecal suspension.
62. The method of embodiment 46 wherein the predetermined percentile rank is an at least 90th percentile rank.
63. The method of any of embodiments 46-61 wherein the predetermined percentile rank is an at least 90th percentile rank.
64. The method of embodiment 46 wherein the cutoff values for males and females of the patient group have a difference of at least 10% (abs).
65. The method of any of embodiments 46-63 wherein the cutoff values for males and females of the patient group have a difference of at least 10% (abs).
66. The method of embodiment 26 or 46, further comprising a normalizing each test result to each patient's total immunoglobulin.
67. The method of any of embodiments 26-65, further comprising a normalizing each test result to each patient's total immunoglobulin.
68. The method of embodiment 26 or 46, further comprising a normalizing each test result to a global mean of each patient's food specific immunoglobulin results.
69. The method of any of embodiments 26-65, further comprising a normalizing each result to the global mean of each patient's food specific immunoglobulin results.
70. The method of embodiment 26 or 46, further comprising an identifying a subset of patients, wherein the subset of patients' sensitivities to the food preparations underlies osteoarthritis by raw p- value or an average discriminatory p-value of ≤ 0.01.
71. The method of any of embodiments 26-65, further comprising an identifying a subset of patients, wherein the subset of patients' sensitivities to the food preparations underlies osteoarthritis by raw p-value or an average discriminatory p-value of ≤ 0.01.
72. The method of embodiment 26 or 46, further comprising a determining numbers of the food preparations, wherein the numbers of the food preparations can be used to confirm osteoarthritis by raw p-value or an average discriminatory p-value of ≤ 0.01.
73. The method of any of embodiments 26-65, further comprising a determining numbers of the food preparations, wherein the numbers of the food preparations can be used to confirm osteoarthritis by raw p-value or an average discriminatory p-value of ≤ 0.01.
74. Use of a plurality of distinct food preparations coupled to individually addressable respective solid carriers in a diagnosis of osteoarthritis, wherein the plurality of distinct food preparations have an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
75. Use of embodiment 74 wherein the plurality of food preparations includes at least two food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
76. Use of embodiment 74 wherein the plurality of food preparations includes at least four food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
77. Use of embodiment 74 wherein the plurality of food preparations includes at least eight food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
78. Use of embodiment 74 wherein the plurality of food preparations includes at least 12 food preparations prepared from food items of Table 1 or selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.
79. Use of embodiment 74 wherein the plurality of distinct food preparations has an average discriminatory p-value of ≤ 0.05 as determined by raw p-value or an average discriminatory p-value of ≤ 0.08 as determined by FDR multiplicity adjusted p-value, and wherein the average discriminatory p-value is determined from a patient test group that is not diagnosed or suspected of having osteoarthritis.
80. Use of any one of embodiments 74-78, wherein the plurality of distinct food preparations has an average discriminatory p-value of ≤ 0.05 as determined by raw p-value or an average discriminatory p-value of ≤ 0.08 as determined by FDR multiplicity adjusted p-value, and wherein the average discriminatory p-value is determined from a patient test group that is not diagnosed or suspected of having osteoarthritis.
81. Use of embodiment 74 wherein the plurality of distinct food preparations has an average discriminatory p-value of ≤ 0.025 as determined by raw p-value or an average discriminatory p-value of ≤ 0.07 as determined by FDR multiplicity adjusted p-value, and wherein the average discriminatory p-value is determined from a patient test group that is not diagnosed or suspected of having osteoarthritis.
82. Use of any one of embodiments 74-78 wherein the plurality of distinct food preparations has an average discriminatory p-value of ≤ 0.025 as determined by raw p-value or an average discriminatory p-value of ≤ 0.07 as determined by FDR multiplicity adjusted p-value, and wherein the average discriminatory p-value is determined from a patient test group that is not diagnosed or suspected of having osteoarthritis.
83. Use of embodiment 74 wherein FDR multiplicity adjusted p-value is adjusted for at least one of age and gender.
84. Use of any one of embodiments 74-82 wherein FDR multiplicity adjusted p-value is adjusted for at least one of age and gender.
85. Use of embodiment 74 wherein FDR multiplicity adjusted p-value is adjusted for age and gender.
86. Use of any one of embodiments 74-82 wherein FDR multiplicity adjusted p-value is adjusted for age and gender.
87. Use of embodiment 74 wherein at least 50% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
88. Use of any one of embodiments 74-86 wherein at least 50% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
89. Use of embodiment 74 wherein at least 70% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
90. Use of any one of the embodiments 74-86 wherein at least 70% of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
91. Use of embodiment 74 wherein all of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p- value, and wherein the average discriminatory p-value is determined from a patient test group that is not diagnosed or suspected of having osteoarthritis.
92. Use of any one of the embodiments 74-86 wherein all of the plurality of distinct food preparations, when adjusted for a single gender, has an average discriminatory p-value of ≤ 0.07 as determined by raw p-value or an average discriminatory p-value of ≤ 0.10 as determined by FDR multiplicity adjusted p-value, and wherein the average discriminatory p-value is determined from a patient test group that is not diagnosed or suspected of having osteoarthritis.
93. Use of embodiment 74 wherein the plurality of distinct food preparations is crude filtered aqueous extracts.
94. Use of any one of the embodiments 74-92 wherein the plurality of distinct food preparations is crude filtered aqueous extracts.
95. Use of embodiment 74 wherein the plurality of distinct food preparations is processed aqueous extracts.
96. Use of any one of the embodiments 74-94 wherein the plurality of distinct food preparations is processed aqueous extracts.
97. Use of embodiment 74 wherein the solid carrier is a well of a multiwall plate, a bead, an electrical sensor, a chemical sensor, a microchip, or an adsorptive film.
98. Use of any one of the embodiments 74-96 wherein the solid carrier is a well of a multiwall plate, a bead, an electrical sensor, a chemical sensor, a microchip, or an adsorptive film.

Thus, it should be appreciated that by having a high-confidence test system as described herein, the rate of false-positive and false negatives can be significantly reduced, and especially where the test systems and methods are gender stratified or adjusted for gender differences as shown below. Such advantages have heretofore not been realized and it is expected that the systems and methods presented herein will substantially increase the predictive power of food sensitivity tests for patients diagnosed with or suspected to have osteoarthritis.

### Experiments

General Protocol for food preparation generation: Commercially available food extracts (available from Biomerica Inc., 17571 Von Karman Ave, Irvine, CA 92614) prepared from the edible portion of the respective raw foods were used to prepare ELISA plates following the manufacturer's instructions.

In certain embodiments, for some food extracts, the inventors found that food extracts prepared with specific procedures to generate food extracts provides more desirable results in detecting elevated IgG reactivity in osteoarthritis patients compared to commercially available food extracts. For example, for grains and nuts, a three-step procedure of generating food extracts is desirable. The first step is a defatting step. In this step, lipids from grains and nuts are extracted by contacting the flour of grains and nuts with a non-polar solvent and collecting residue. Then, the defatted grain or nut flour are extracted by contacting the flour with elevated pH to obtain a mixture and removing the solid from the mixture to obtain the liquid extract. Once the liquid extract is generated, the liquid extract is stabilized by adding an aqueous formulation. In one embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

For another example, for meats and fish, a two-step procedure of generating food extract is desirable in certain embodiments. The first step is an extraction step. In this step, extracts from raw, uncooked meats or fish are generated by emulsifying the raw, uncooked meats or fish in an aqueous buffer formulation in a high impact pressure processor. Then, solid materials are removed to obtain liquid extract. Once the liquid extract is generated, the liquid extract is stabilized by adding an aqueous formulation. In one embodiment, the aqueous formulation includes a sugar alcohol, a metal chelating agent, protease inhibitor, mineral salt, and buffer component 20-50 mM of buffer from 4-9 pH. This formulation allowed for long term storage at - 70 °C and multiple freeze-thaws without a loss of activity.

For still another example, for fruits and vegetables, a two step procedure of generating food extract is desirable. The first step is an extraction step. In this step, liquid extracts from fruits or vegetables are generated using an extractor (e.g., masticating juicer, etc) to pulverize foods and extract juice. Then, solid materials are removed to obtain liquid extract. Once the liquid extract is generated, the liquid extract is stabilized by adding an aqueous formulation. In one embodiment, the aqueous formulation includes a sugar alcohol, a metal chelating agent, protease inhibitor, mineral salt, and buffer component 20-50 mM of buffer from 4-9 pH. This formulation allowed for long term storage at -70 °C and multiple freeze-thaws without a loss of activity.

Blocking of ELISA plates: To optimize signal to noise, plates were blocked with a proprietary blocking buffer. In a certain embodiments, the blocking buffer includes 20-50 mM of buffer from 4-9 pH, a protein of animal origin *(e.g.,* beef, chicken) and a short chain alcohol. *(e.g.,* glycerin) Other blocking buffers, including several commercial preparations that did not meet the foregoing criteria, were attempted for use but could not provide adequate signal to noise and low assay variability that was desired.

ELISA preparation and sample testing: Food antigen preparations were immobilized onto respective microtiter wells following the manufacturer's instructions. For the assays, the food antigens were allowed to react with antibodies present in the patients' serum, and excess serum proteins were removed by a wash step. For detection of IgG antibody binding, enzyme labeled anti-IgG antibody conjugate was allowed to react with antigen-antibody complex. A color was developed by the addition of a substrate that reacts with the coupled enzyme. The color intensity was measured and is directly proportional to the concentration of IgG antibody specific to a particular food antigen.

Methodology to determine ranked food list in order of ability of ELISA signals to distinguish osteoarthritis from control subjects: Out of an initial selection *(e.g.,* 100 food items, or 150 food items, or even more), samples can be eliminated prior to analysis due to low consumption in an intended population. In addition, specific food items can be used as being representative of a larger more generic food group, especially where prior testing has established a correlation among different species within a generic group (in both genders in some embodiments, but also suitable for correlation for a single gender in other embodiments). For example, chili pepper could be dropped in favor of green pepper as representative of the "pepper" food group, or cheddar cheese could be dropped in favor of American cheese as representative of the "cheese" food group. In further certain embodiments, the final list of foods is shorter than 50 food items, and, in certain embodiments, equal or less than 40 food items.

Since the foods ultimately selected for the food intolerance panel will not be specific for a particular gender, a gender-neutral food list was .desirable in certain embodiments Since the observed sample was imbalanced by gender *(e.g.,* controls: 72% female, osteoarthritis: 68% female), differences in ELISA signal magnitude strictly due to gender were removed for certain embodiments by modeling signal scores against gender using a two-sample t-test and storing the residuals for further analysis. For each of those tested foods, residual signal scores were compared between osteoarthritis and controls using a permutation test on a two-sample t-test with 50,000 resamplings. The Satterthwaite approximation was used for the denominator degrees of freedom to account for lack of homogeneity of variances, and the 2-tailed permuted p-value represented the raw p-value for each food. False Discovery Rates (FDR) among the comparisons, were adjusted by any acceptable statistical procedures (e.g., Benjamini-Hochberg, Family-wise Error Rate (FWER), Per Comparison Error Rate (PCER), etc.).

Foods were then ranked according to their 2-tailed FDR multiplicity-adjusted p-values. Foods with adjusted p-values equal to or lower than the desired FDR threshold were deemed to have significantly higher signal scores among osteoarthritis than control subjects and therefore deemed candidates for inclusion into a food intolerance panel. A typical result that is representative of the outcome of the statistical procedure is provided in **Table 2.** Here the ranking of foods is according to 2-tailed permutation T-test p-values with FDR adjustment.

Notably in certain embodiments, the inventors discovered that even for the same food preparation tested, the ELISA score for at least several food items varied dramatically, and exemplary raw data are provided in **Table 3.** As will be readily appreciated, in certain embodiments, data unstratified by gender will therefore lose significant explanatory power where the same cutoff value is applied to raw data for male and female data. To overcome such disadvantage in such embodiments, the inventors stratified the data by gender as described below.

Statistical Method for Cutpoint Selection for each Food: The determination of what ELISA signal scores would constitute a "positive" response was made by summarizing the distribution of signal scores among the Control subjects. In certain embodiments, for each food, osteoarthritis subjects who had have observed scores greater than or equal to selected quantiles of the Control subject distribution were deemed "positive". To attenuate the influence of any one subject on cutpoint determination, each food-specific and gender-specific dataset was bootstrap resampled 1000 times. Within each bootstrap replicate, the 90th and 95th percentiles of the Control signal scores were determined. Each osteoarthritis subject in the bootstrap sample was compared to the 90th and 95% percentiles to determine whether he/she had a "positive" response. The final 90th and 95th percentile-based cutpoints for each food and gender were computed as the average 90th and 95th percentiles across the 1000 samples. The number of foods for which each osteoarthritis subject was rated as "positive" was computed by pooling data across foods. Using such method, the inventors were now able to identify cutoff values for a predetermined percentile rank that in most cases was substantially different as can be taken from **Table 4.**

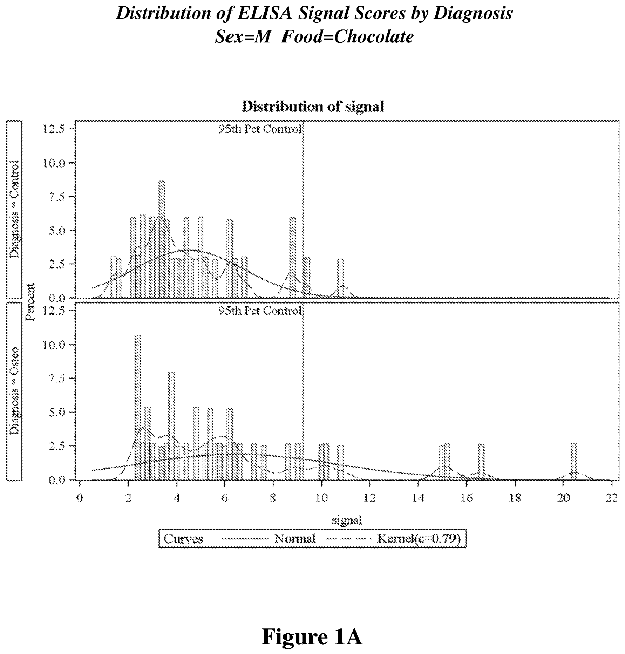

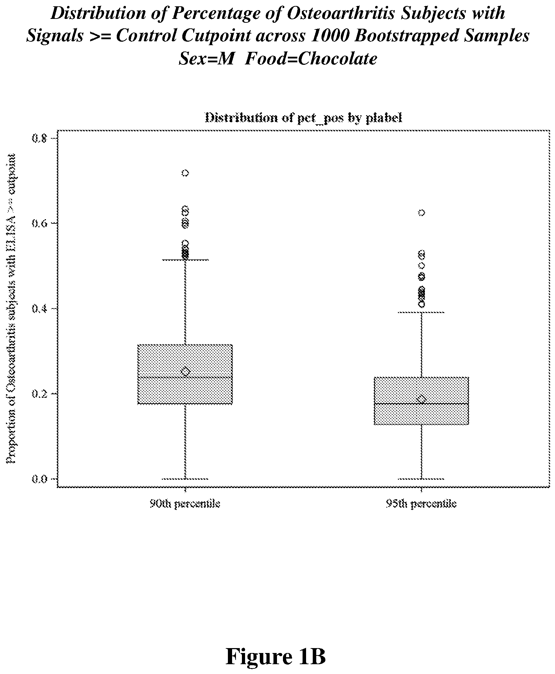

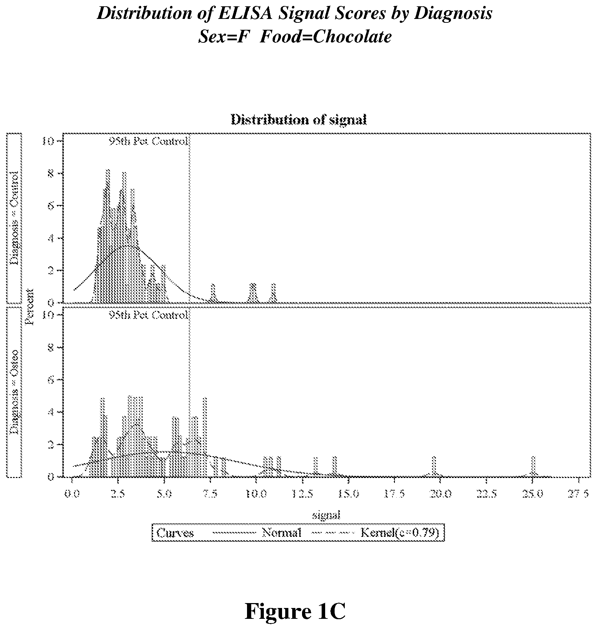

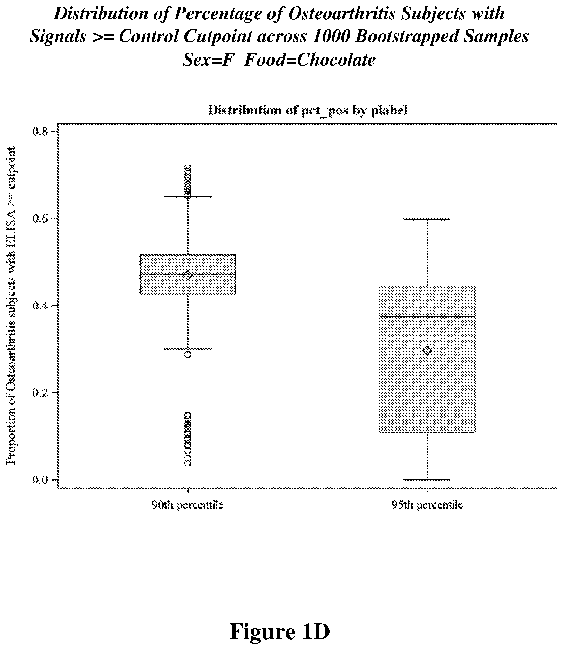

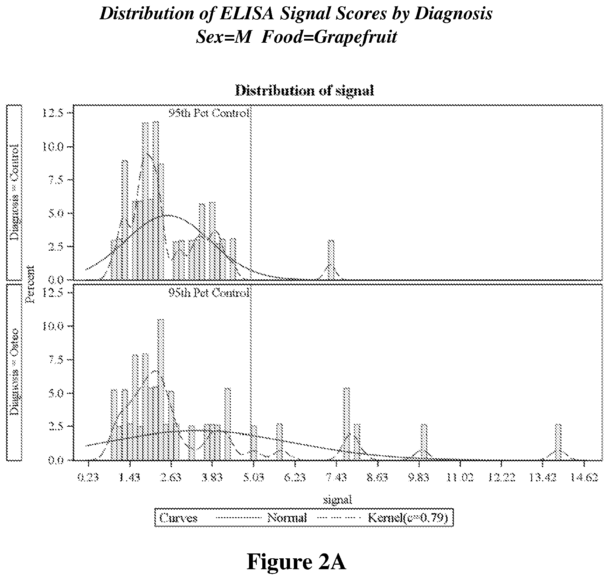

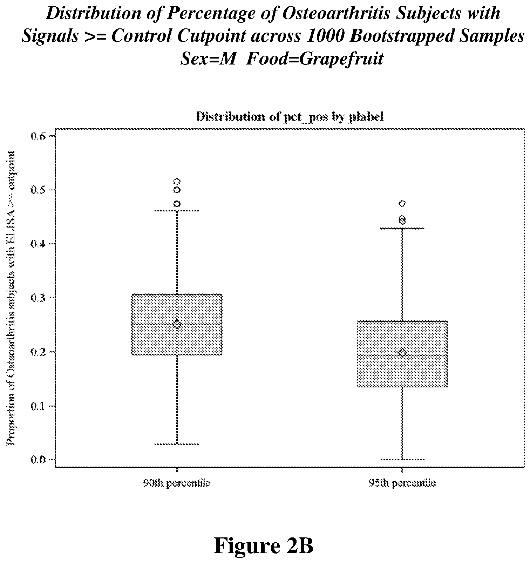

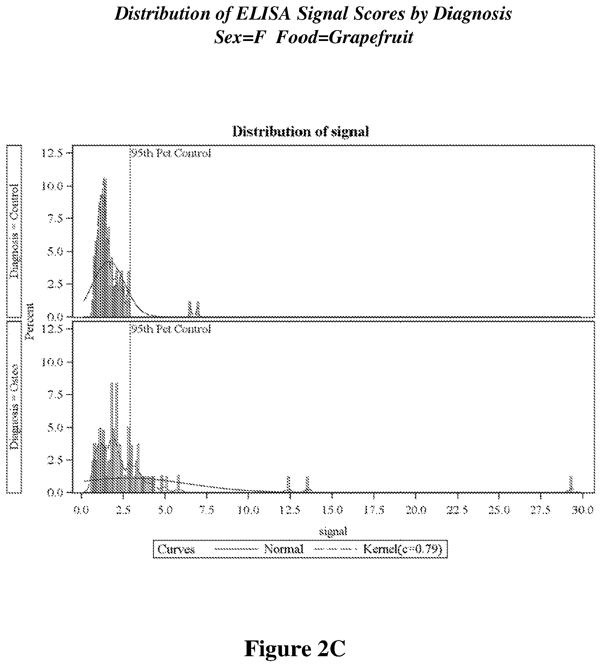

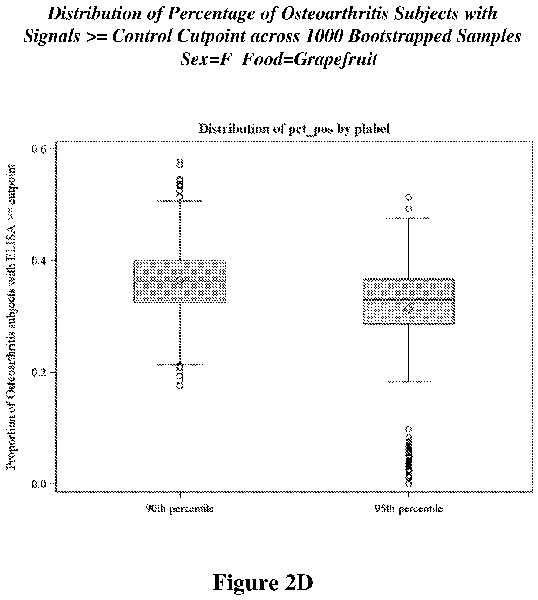

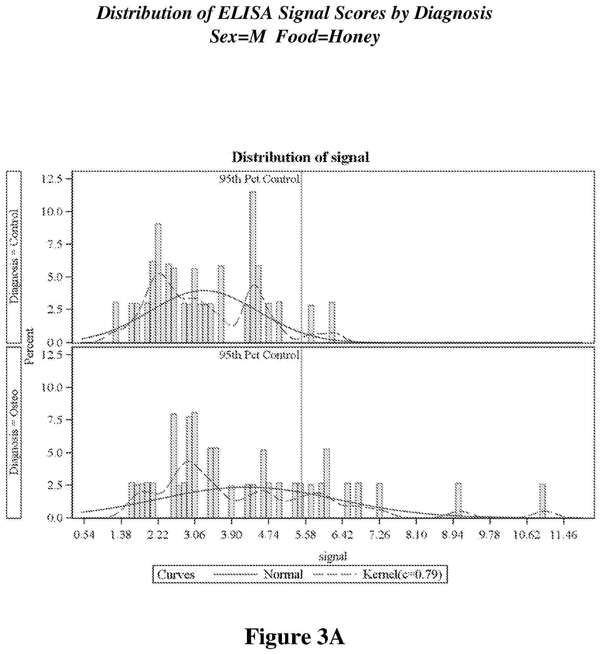

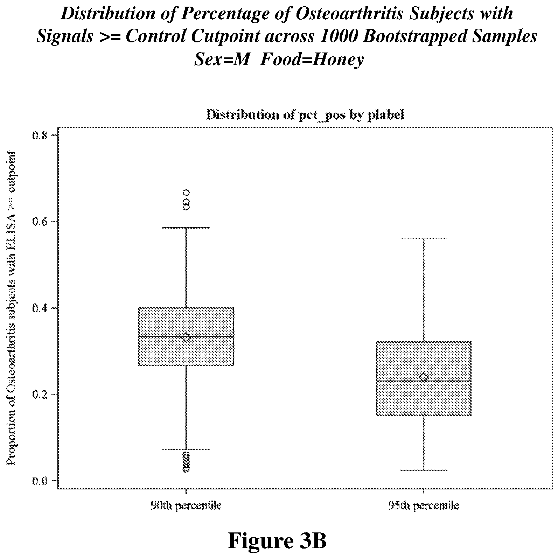

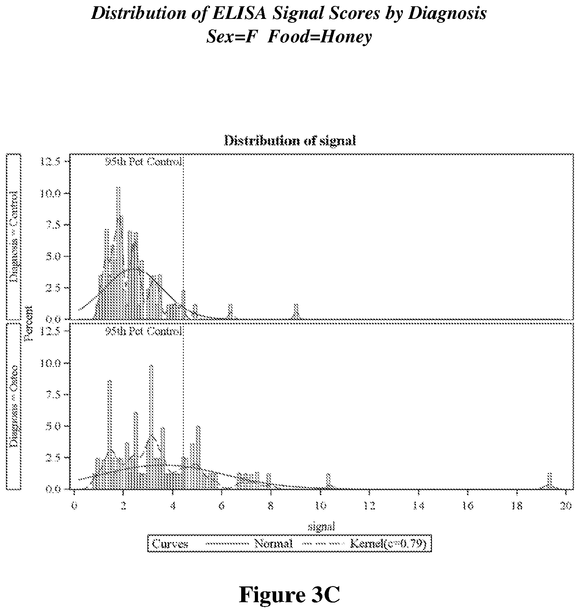

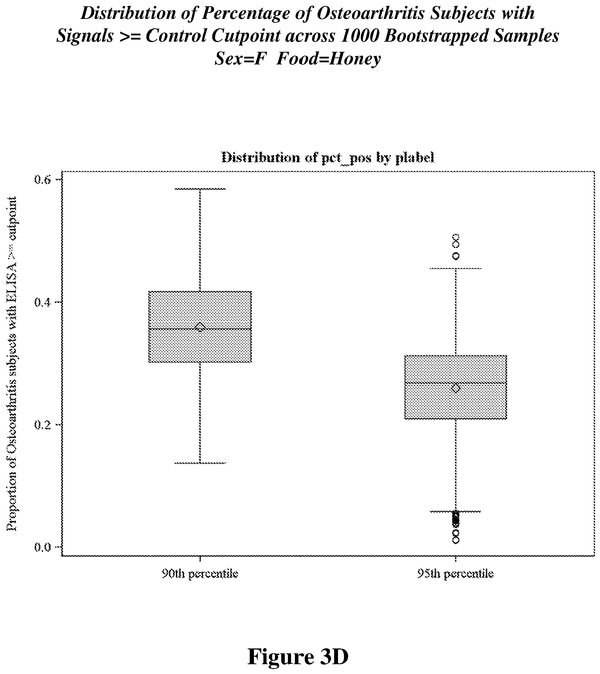

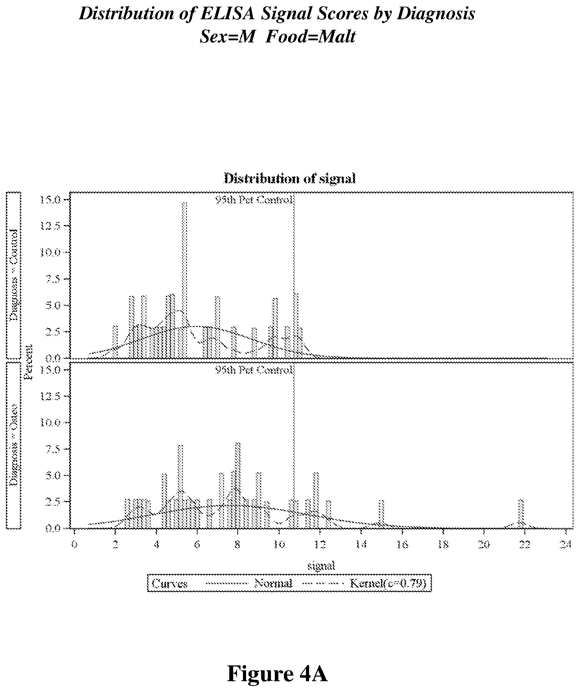

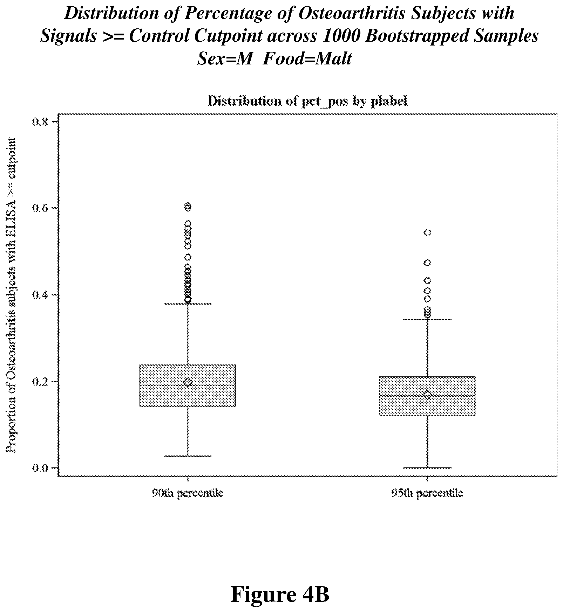

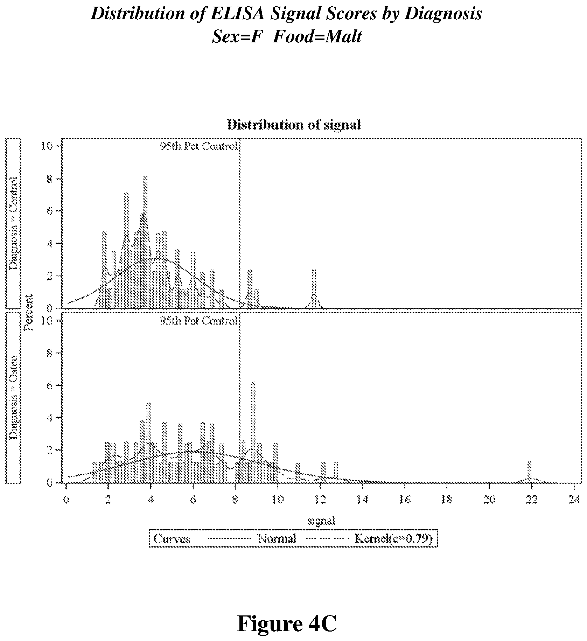

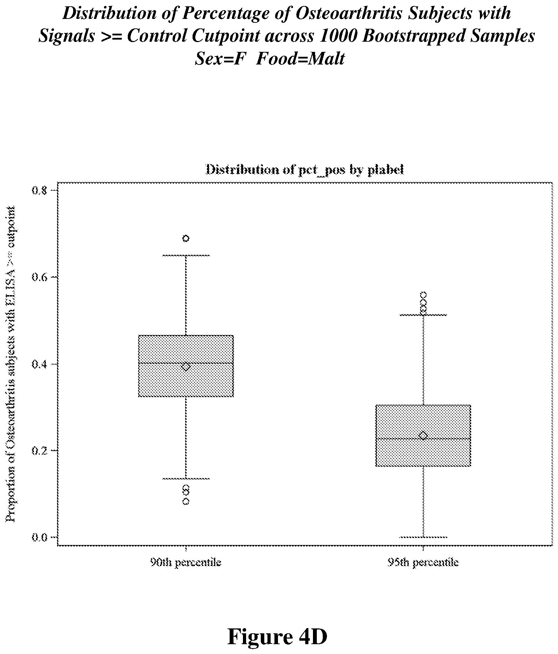

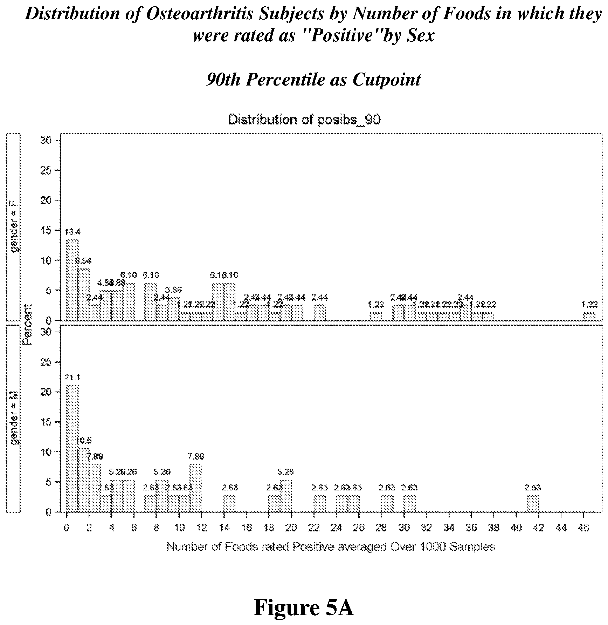

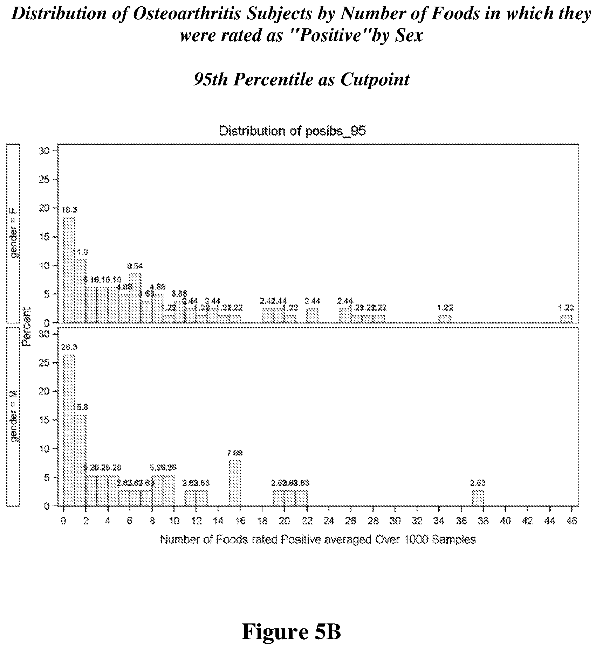

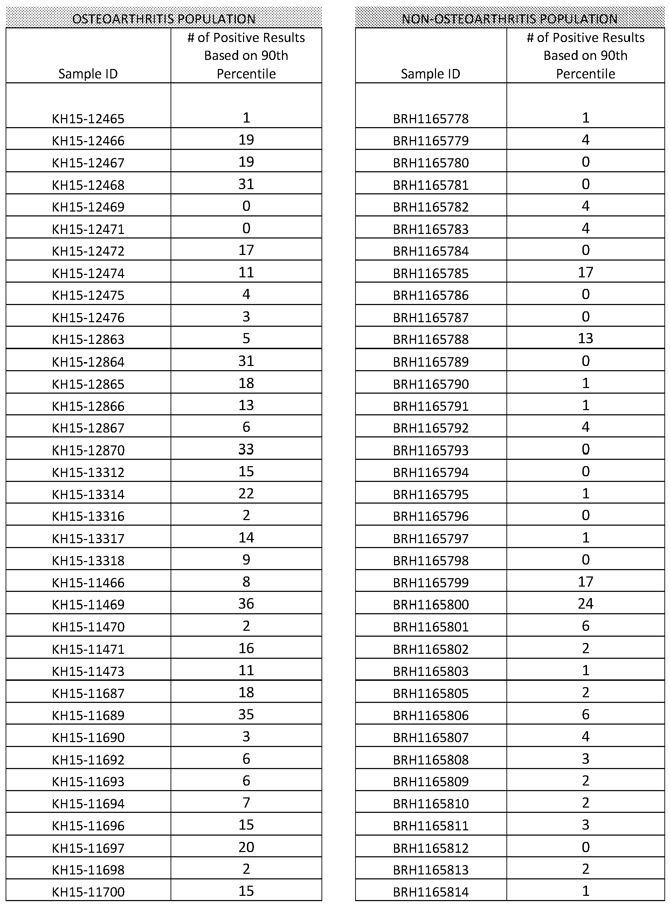

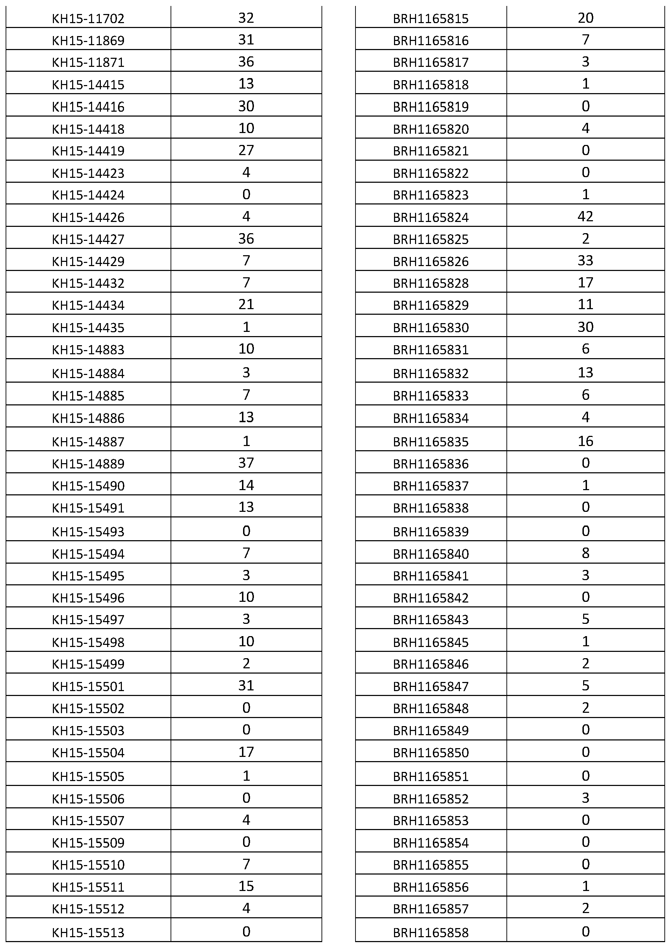

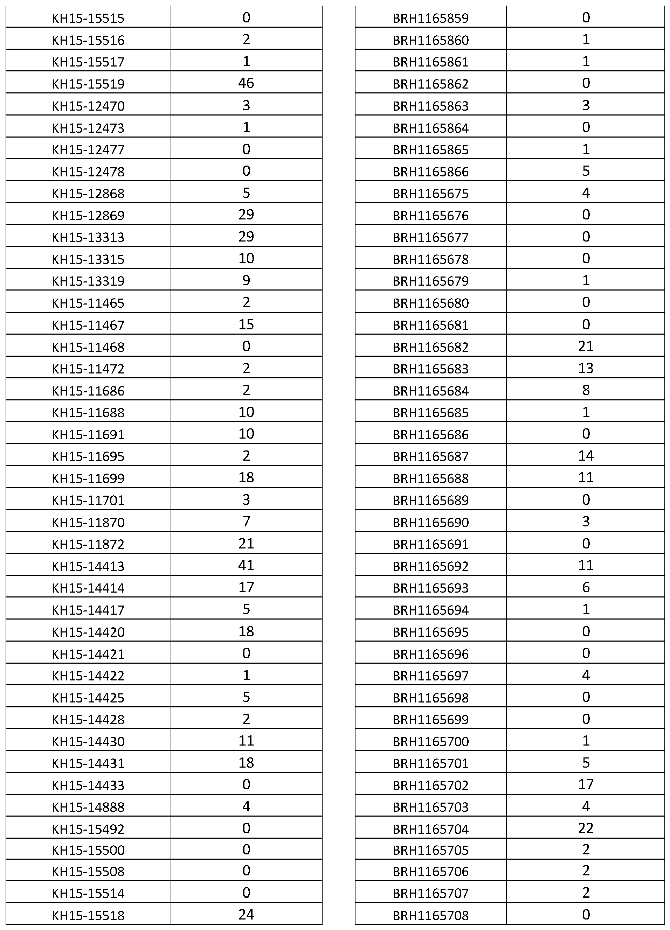

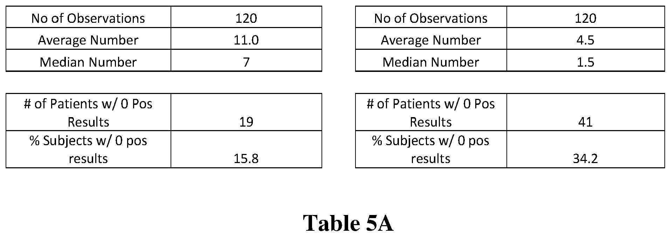

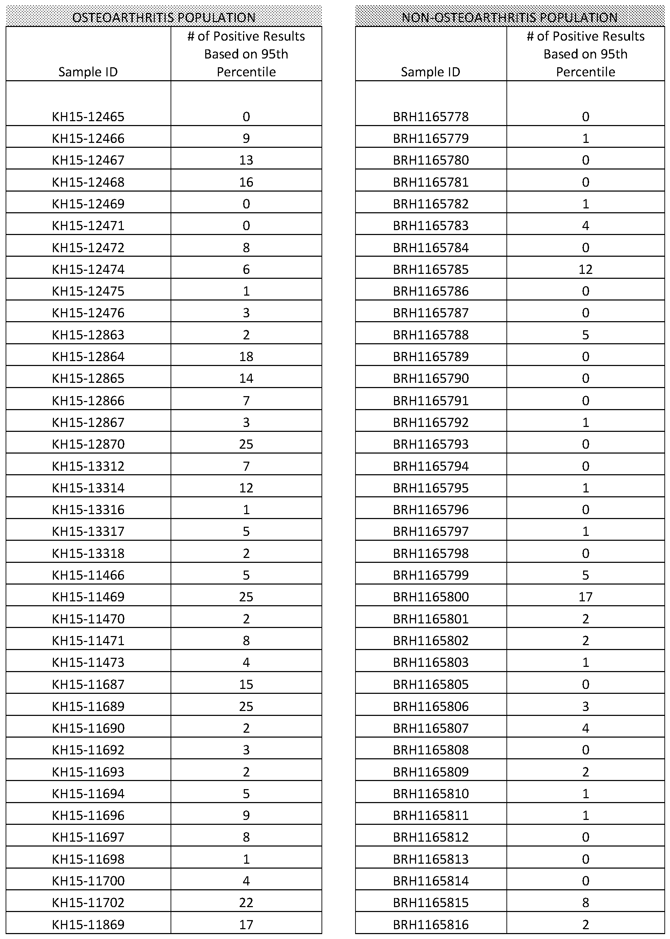

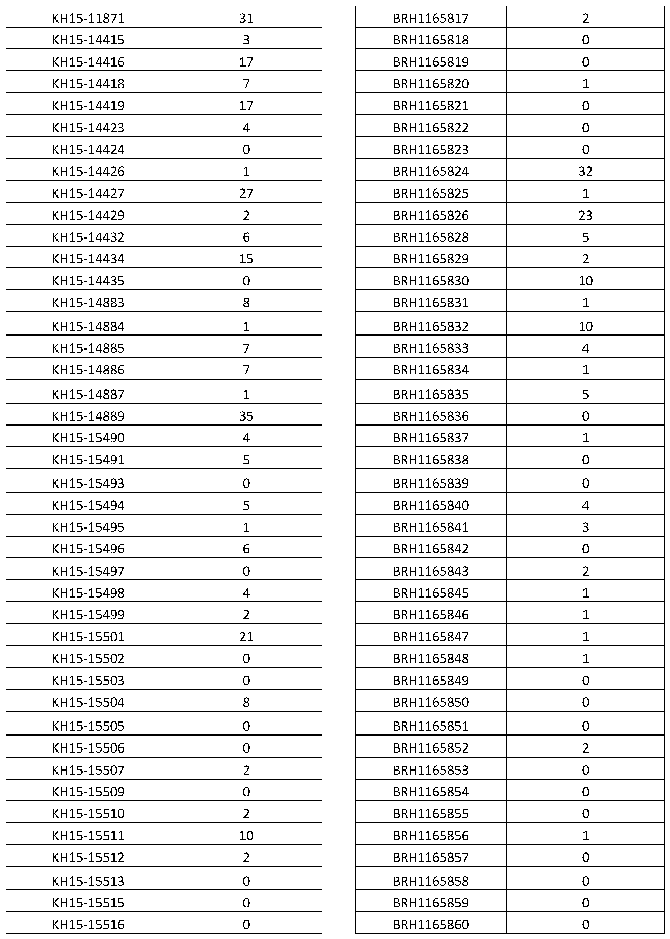

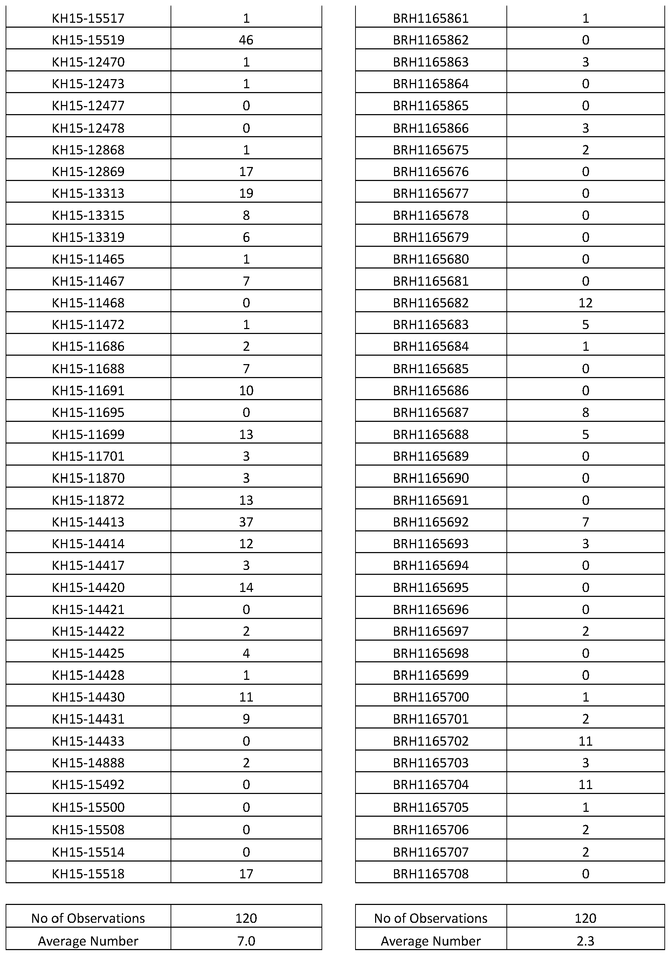

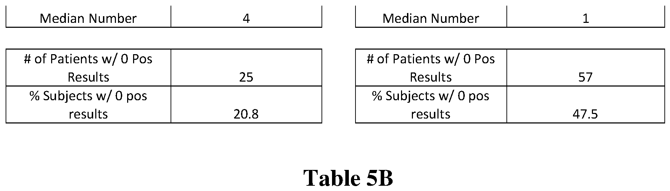

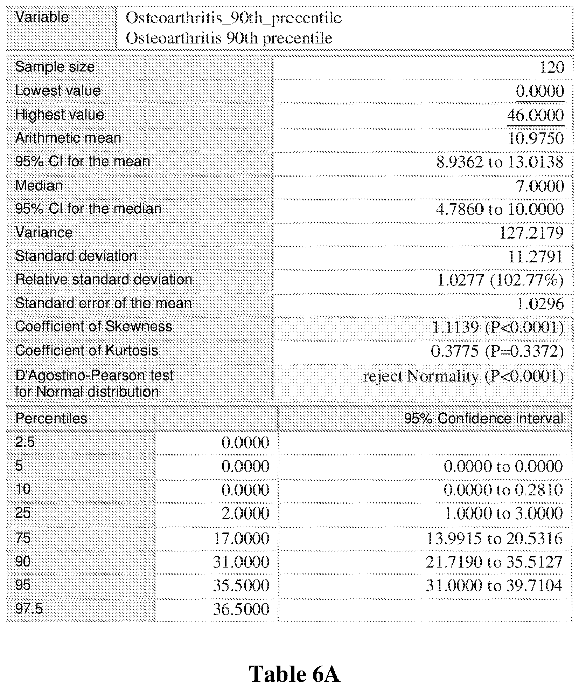

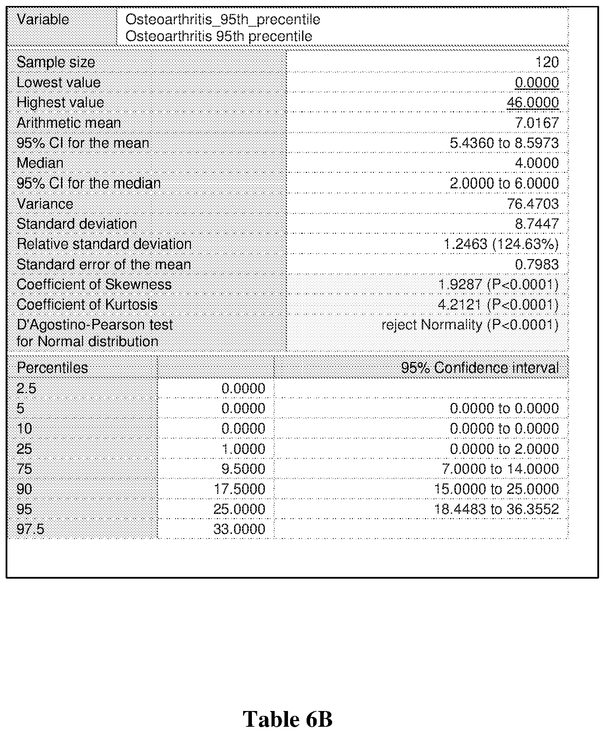

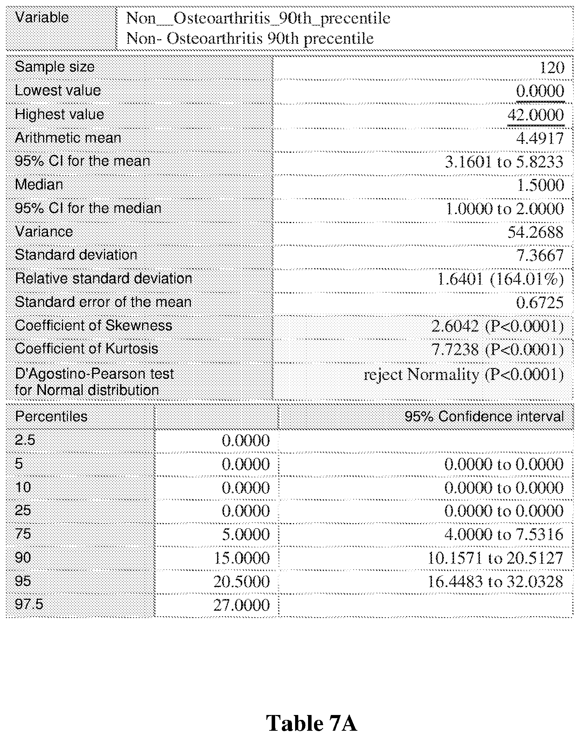

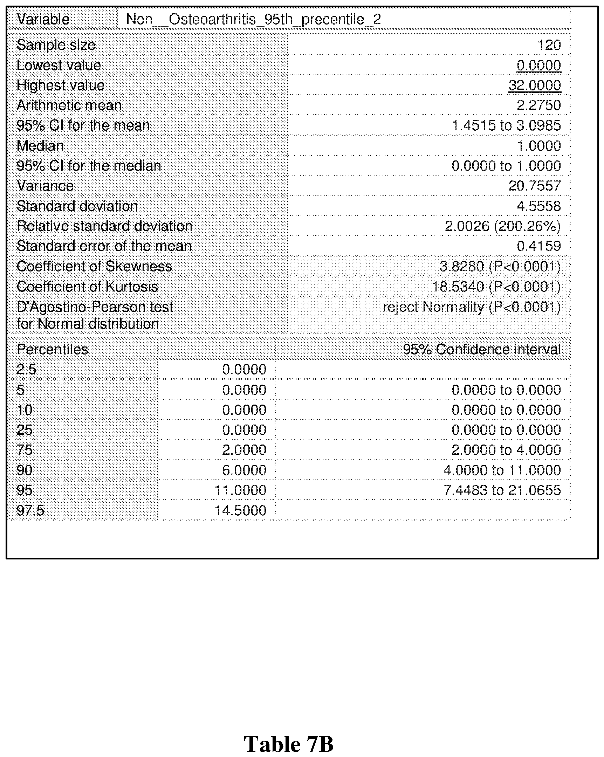

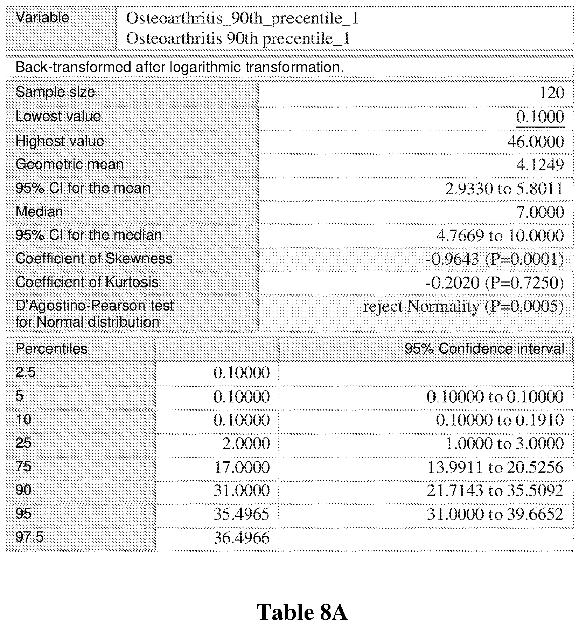

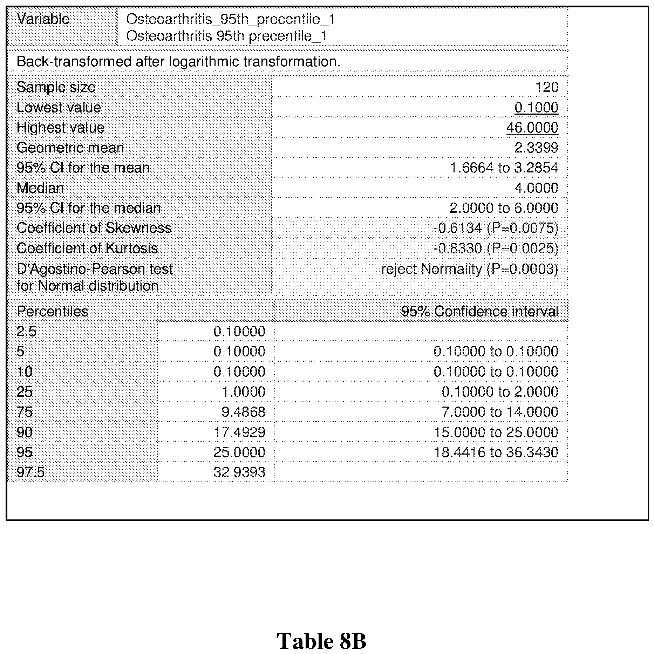

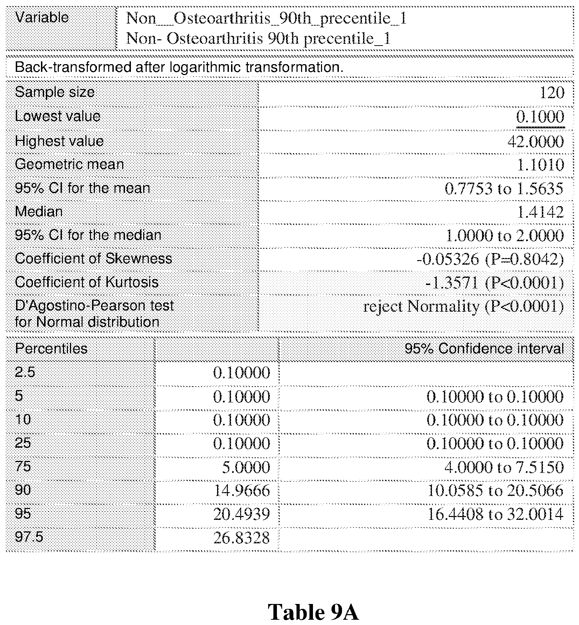

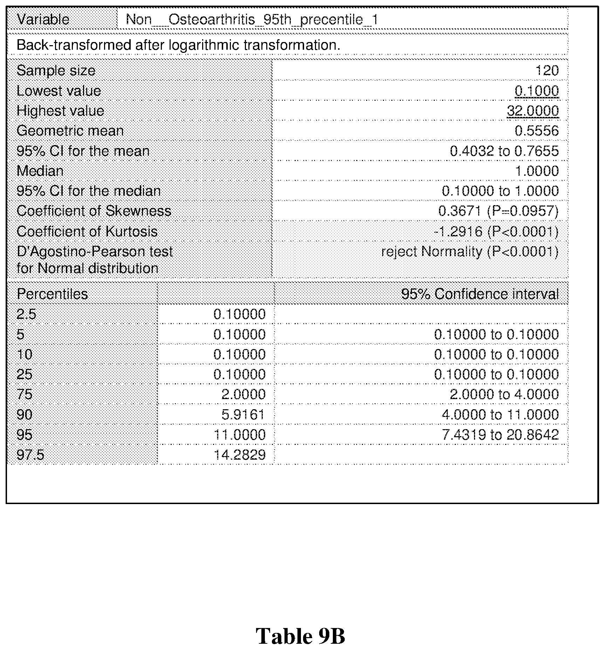

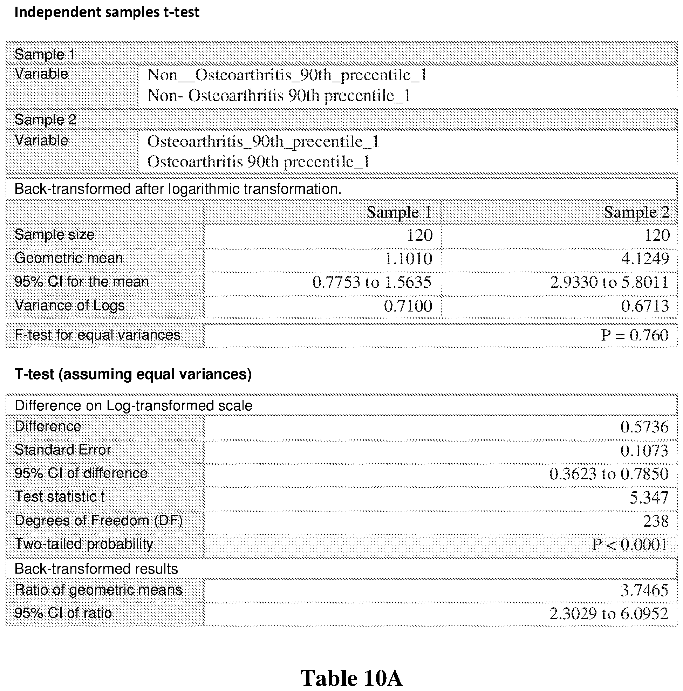

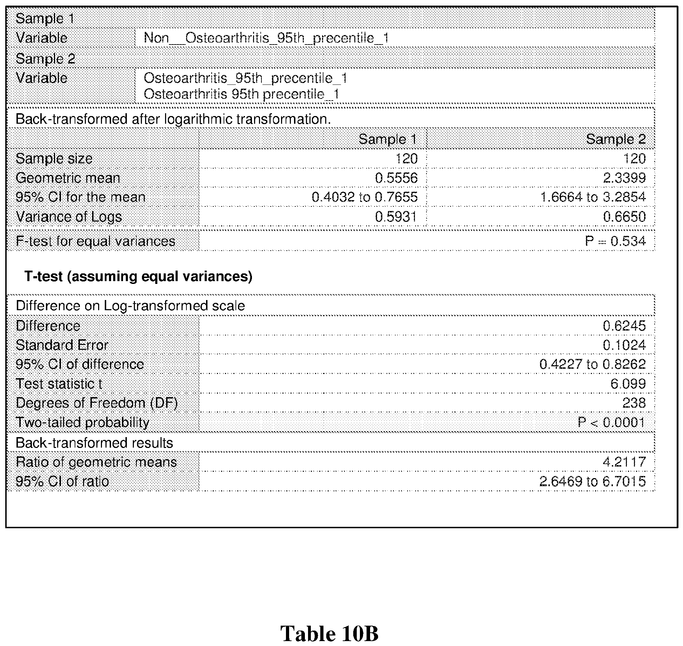

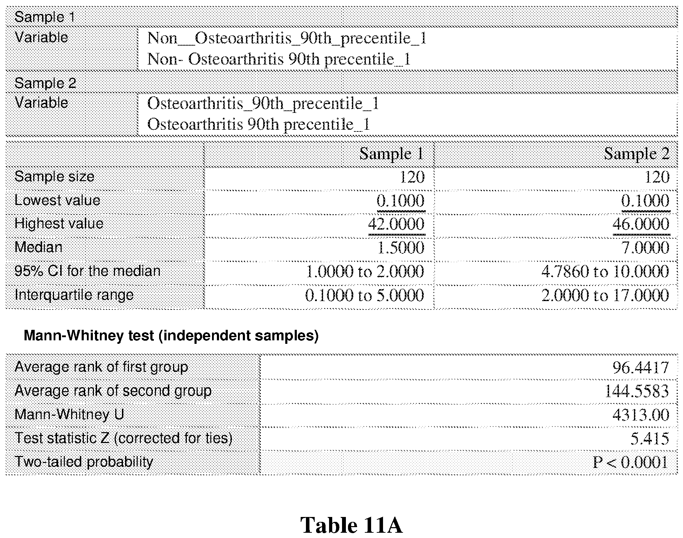

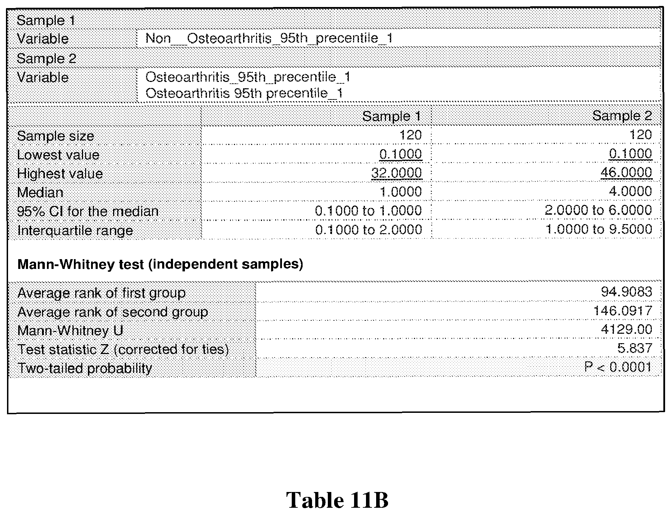

Typical examples for the gender difference in IgG response in blood with respect to chocolate is shown in **Figures 1A-1D****,** where **Figure 1A** shows the signal distribution in men along with the 95^{th} percentile cutoff as determined from the male control population. **Figure 1B** shows the distribution of percentage of male osteoarthritis subjects exceeding the 90^{th} and 95^{th} percentile, while **Figure 1C** shows the signal distribution in women along with the 95^{th} percentile cutoff as determined from the female control population. **Figure 1D** shows the distribution of percentage of female osteoarthritis subjects exceeding the 90^{th} and 95^{th} percentile. In the same fashion, **Figures 2A-2D** exemplarily depict the differential response to grapefruit, **Figures 3A-3D** exemplarily depict the differential response to honey, and **Figures 4A-4D** exemplarily depict the differential response to malt. **Figures 5A-5B** show the distribution of osteoarthritis subjects by number of foods that were identified as trigger foods at the 90^{th} percentile (5A) and 95^{th} percentile (5B). Inventors contemplate that regardless of the particular food items, male and female responses were notably distinct.

It should be noted that nothing in the art has provided any predictable food groups related to osteoarthritis that is gender-stratified. Thus, a discovery of food items that show distinct responses by gender is a surprising result, which was not expected by the inventors. In other words, selection of food items based on gender stratification provides an unexpected technical effect such that statistical significances for particular food items as triggering food among male or female osteoarthritis patients have been significantly improved.

Normalization of IgG Response Data: While the raw data of the patient's IgG response results can be use to compare strength of response among given foods, it is also contemplated that the IgG response results of a patient are normalized and indexed to generate unit-less numbers for comparison of relative strength of response to a given food. For example, one or more of a patient's food specific IgG results *(e.g.,* IgG specific to malt and IgG specific to grapefruit) can be normalized to the patient's total IgG. The normalized value of the patient's IgG specific to malt can be 0.1 and the normalized value of the patient's IgG specific to grapefruit can be 0.3. In this scenario, the relative strength of the patient's response to grapefruit is three times higher compared to malt. Then, the patient's sensitivity to grapefruit and malt can be indexed as such.

In other examples, one or more of a patient's food specific IgG results *(e.g.,* IgG specific to lobster and IgG specific to pork) can be normalized to the global mean of that patient's food specific IgG results. The global means of the patient's food specific IgG can be measured by total amount of the patient's food specific IgG. In this scenario, the patient's specific IgG to lobster can be normalized to the mean of patient's total food specific IgG (*e.g.,* mean of IgG levels to lobster, pork, Dungeness crab, chicken, peas, etc.). However, it is also contemplated that the global means of the patient's food specific IgG can be measured by the patient's IgG levels to a specific type of food via multiple tests. If the patient have been tested for his sensitivity to lobster five times and to pork seven times previously, the patient's new IgG values to lobster or to pork are normalized to the mean of five-times test results to lobster or the mean of seven-times test results to pork. The normalized value of the patient's IgG specific to lobster can be 6.0 and the normalized value of the patient's IgG specific to pork can be 1.0. In this scenario, the patient has six times higher sensitivity to lobster at this time compared to his average sensitivity to lobster, but substantially similar sensitivity to pork. Then, the patient's sensitivity to lobster and pork can be indexed based on such comparison.

Methodology to determine the subset of osteoarthritis patients with food sensitivities that underlie osteoarthritis: While it is suspected that food sensitivities may play a substantial role in signs and symptoms of osteoarthritis, some osteoarthritis patients may not have food sensitivities that underlie osteoarthritis. Those patients would not benefit from dietary intervention to treat signs and symptoms of osteoarthritis. To determine the subset of such patients, body fluid samples of osteoarthritis patients and non- osteoarthritis patients can be tested with an ELISA test using test devices with 24 food samples.

**Table 5A** and **Table 5B** provide exemplary raw data. As should be readily appreciated, data indicates number of positive results out of 90 sample foods based on 90^{th} percentile value (Table 5A) or 95^{th} percentile value (Table 5B). First column is Osteoarthritis (n=120); second column is non-Osteoarthritis (n=120) by ICD-10 code. Average and median number of positive foods was computed for Osteoarthritis and non-Osteoarthritis patients. From the raw data shown in Table 5A and Table 5B, average and standard deviation of the number of positive foods was computed for osteoarthritis and non-osteoarthritis patients. Additionally, the number and percentage of patients with zero positive foods was calculated for both osteoarthritis and non-osteoarthritis. The number and percentage of patients in the osteoarthritis population with zero positive foods is less than half of that found in the non-osteoarthritis population (15.8% vs. 34.2%, respectively) based on 90^{th} percentile value (Table 5A), and based on 95^{th} percentile value the number and percentage of patients in the osteoarthritis population with zero positive foods is also less than half of that found in the non-osteoarthritis (20.8% vs. 47.5%, respectively (Table 5B). Thus, it can be easily appreciated that the osteoarthritis patient having sensitivity to zero positive foods is unlikely to have food sensitivities underlying their signs and symptoms of osteoarthritis.

**Table 6A** and **Table 7A** show exemplary statistical data summarizing the raw data of two patient populations shown in Table 5A. The statistical data includes normality, arithmetic mean, median, percentiles and 95% confidence interval (CI) for the mean and median representing number of positive foods in the osteoarthritis population and the non-osteoarthritis population. **Table 6B** and **Table 7B** show exemplary statistical data summarizing the raw data of two patient populations shown in Table 5B. The statistical data includes normality, arithmetic mean, median, percentiles and 95% confidence interval (CI) for the mean and median representing number of positive foods in the osteoarthritis population and the non-osteoarthritis population.

**Table 8A** and **Table 9A** show another exemplary statistical data summarizing the raw data of two patient populations shown in Table 5A. In Tables 8A and 9A, the raw data was transformed by logarithmic transformation to improve the data interpretation. **Table 8B** and **Table 9B** show another exemplary statistical data summarizing the raw data of two patient populations shown in Table 5B. In Tables 8B and 9B, the raw data was transformed by logarithmic transformation to improve the data interpretation.

**Table 10A** and **Table 11A** show exemplary statistical data of an independent T-test (Table 10A, logarithmically transformed data) and a Mann-Whitney test (Table 11A) to compare the geometric mean number of positive foods between the osteoarthritis and non-osteoarthritis samples. The data shown in Table 10A and Table 11A indicates statistically significant differences in the geometric mean of positive number of foods between the osteoarthritis population and the non-osteoarthritis population. In both statistical tests, it is shown that the number of positive responses with 90 food samples is significantly higher in the osteoarthritis population than in the non-osteoarthritis population with an average discriminatory p-value of ≤ 0.0001. These statistical data is also illustrated as a box and whisker plot in **Figure 6A****,** and a notched box and whisker plot in **Figure 6B****.**

**Table 10B** and **Table 11B** show exemplary statistical data of an independent T-test (Table 10A, logarithmically transformed data) and a Mann-Whitney test (Table 11B) to compare the geometric mean number of positive foods between the osteoarthritis and non-osteoarthritis samples. The data shown in Table 10B and Table 11B indicates statistically significant differences in the geometric mean of positive number of foods between the osteoarthritis population and the non-osteoarthritis population. In both statistical tests, it is shown that the number of positive responses with 90 food samples is significantly higher in the osteoarthritis population than in the non-osteoarthritis population with an average discriminatory p-value of ≤ 0.0001. These statistical data is also illustrated as a box and whisker plot in **Figure 6C**, and a notched box and whisker plot in **Figure 6D**.

**Table 12A** shows exemplary statistical data of a Receiver Operating Characteristic (ROC) curve analysis of data shown in Tables 5A-11A to determine the diagnostic power of the test used in Table 5 at discriminating osteoarthritis from non- osteoarthritis subjects. When a cutoff criterion of more than 6 positive foods is used, the test yields a data with 72.4% sensitivity and 72.2% specificity, with an area under the curve (AUROC) of 0.771. The p-value for the ROC is significant at a p-value of <0.0001. **Figure 7A** illustrates the ROC curve corresponding to the statistical data shown in Table 12A. Because the statistical difference between the osteoarthritis population and the non-osteoarthritis population is significant when the test results are cut off to positive number of 6, the number of foods that a patient tests positive could be used as a confirmation of the primary clinical diagnosis osteoarthritis, and whether it is likely that food sensitivities underlies on the patient's signs and symptoms of osteoarthritis. Therefore, the above test can be used as another 'rule in' test to add to currently available clinical criteria for diagnosis for osteoarthritis.

As shown in Tables 5A-12A, and Figure 7A, based on 90^{th} percentile data, the number of positive foods seen in osteoarthritis vs. non-osteoarthritis subjects is significantly different whether the geometric mean or median of the data is compared. The number of positive foods that a person has is indicative of the presence of osteoarthritis in subjects. The test has discriminatory power to detect osteoarthritis with -53% sensitivity and -81% specificity. Additionally, the absolute number and percentage of subjects with 0 positive foods is also very different in osteoarthritis vs. non-osteoarthritis subjects, with a far lower percentage of osteoarthritis subjects (16%) having 0 positive foods than non- osteoarthritis subjects (34%). The data suggests a subset of osteoarthritis patients may have osteoarthritis due to other factors than diet, and may not benefit from dietary restriction.

**Table 12B** shows exemplary statistical data of a Receiver Operating Characteristic (ROC) curve analysis of data shown in Tables 5B-11B to determine the diagnostic power of the test used in Table 5 at discriminating osteoarthritis from non- osteoarthritis subjects. When a cutoff criterion of more than 6 positive foods is used, the test yields a data with 67% sensitivity and 65% specificity, with an area under the curve (AUROC) of 0.713. The p-value for the ROC is significant at a p-value of <0.0001. **Figure 7B** illustrates the ROC curve corresponding to the statistical data shown in Table 12B. Because the statistical difference between the osteoarthritis population and the non-osteoarthritis population is significant when the test results are cut off to positive number of 6, the number of foods that a patient tests positive could be used as a confirmation of the primary clinical diagnosis osteoarthritis, and whether it is likely that food sensitivities underlies on the patient's signs and symptoms of osteoarthritis. Therefore, the above test can be used as another 'rule in' test to add to currently available clinical criteria for diagnosis for osteoarthritis.

As shown in Tables 5B-12B, and Figure 7B, based on 95^{th} percentile data, the number of positive foods seen in osteoarthritis vs. non-osteoarthritis subjects is significantly different whether the geometric mean or median of the data is compared. The number of positive foods that a person has is indicative of the presence of osteoarthritis in subjects. The test has discriminatory power to detect osteoarthritis with -67% sensitivity and -65% specificity. Additionally, the absolute number and percentage of subjects with 0 positive foods is also very different in osteoarthritis vs. non-osteoarthritis subjects, with a far lower percentage of osteoarthritis subjects (20%) having 0 positive foods than non- osteoarthritis subjects (48%). The data suggests a subset of osteoarthritis patients may have osteoarthritis due to other factors than diet, and may not benefit from dietary restriction.

Method for determining distribution of per-person number of foods declared "positive": To determine the distribution of number of "positive" foods per son and measure the diagnostic performance, the analysis was performed with 90 food items from the Table 1, which shows most positive responses to osteoarthritis patients. The 90 food items includes chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, halibut, cabbage, orange, rice, safflower, tomato, almond, oat, barley, peach, grape, potato, spinach, sole, and butter. To attenuate the influence of any one subject on this analysis, each food-specific and gender-specific dataset was bootstrap resampled 1000 times. Then, for each food item in the bootstrap sample, sex-specific cutpoint was determined using the 90th and 95th percentiles of the control population. Once the sex-specific cutpoints were determined, the sex-specific cutpoints was compared with the observed ELISA signal scores for both control and osteoarthritis subjects. In this comparison, if the observed signal is equal or more than the cutpoint value, then it is determined "positive" food, and if the observed signal is less than the cutpoint value, then it is determined "negative" food.

Once all food items were determined either positive or negative, the results of the 180 (90 foods x 2 cutpoints) calls for each subject were saved within each bootstrap replicate. Then, for each subject, 90 calls were summed using 90^{th} percentile as cutpoint to get "Number of Positive Foods (90^{th})," and the rest of 90 calls were summed using 95^{th} percentile to get "Number of Positive Foods (95^{th})." Then, within each replicate, "Number of Positive Foods (90^{th})" and "Number of Positive Foods (95^{th})" were summarized across subjects to get descriptive statistics for each replicate as follows: 1) overall means equals to the mean of means, 2) overall standard deviation equals to the mean of standard deviations, 3) overall medial equals to the mean of medians, 4) overall minimum equals to the minimum of minimums, and 5) overall maximum equals to maximum of maximum. In this analysis, to avoid non-integer "Number of Positive Foods" when computing frequency distribution and histogram, the authors pretended that the 1000 repetitions of the same original dataset were actually 999 sets of new subjects of the same size added to the original sample. Once the summarization of data is done, frequency distributions and histograms were generated for both "Number of Positive Foods (90^{th})" and "Number of Positive Foods (95^{th})" for both genders and for both osteoarthritis subjects and control subjects using programs "a_pos_foods.sas, a_pos_foods_by_dx.sas".

Method for measuring diagnostic performance: To measure diagnostic performance for each food items for each subject, we used data of "Number of Positive Foods (90^{th})" and "Number of Positive Foods (95^{th})" for each subject within each bootstrap replicate described above. In this analysis, the cutpoint was set to 1. Thus, if a subject has one or more "Number of Positive Foods (90^{th})", then the subject is called "Has osteoarthritis." If a subject has less than one "Number of Positive Foods (90^{th})", then the subject is called "Does Not Have osteoarthritis." When all calls were made, the calls were compared with actual diagnosis to determine whether a call was a True Positive (TP), True Negative (TN), False Positive (FP), or False Negative (FN). The comparisons were summarized across subjects to get the performance metrics of sensitivity, specificity, positive predictive value, and negative predictive value for both "Number of Positive Foods(90^{th})" and "Number of Positive Foods(95^{th})" when the cutpoint is set to 1 for each method. Each (sensitivity, 1-specificity) pair becomes a point on the ROC curve for this replicate.

To increase the accuracy, the analysis above was repeated by incrementing cutpoint from 2 up to 24, and repeated for each of the 1000 bootstrap replicates. Then the performance metrics across the 1000 bootstrap replicates were summarized by calculating averages using a program "t_pos_foods_by_dx.sas". The results of diagnostic performance for female and male are shown in Table 13 (90^{th} percentile) and Table 14 (95^{th} percentile).

Of course, it should be appreciated that certain variations in the food preparations may be made without altering the general scope of the subject matter presented herein. For example, where the food item was yellow onion, that item should be understood to also include other onion varieties that were demonstrated to have equivalent activity in the tests. Indeed, the inventors have noted that for each tested food preparation, certain other related food preparations also tested in the same or equivalent manner (data not shown). Thus, it should be appreciated that each tested and claimed food preparation will have equivalent related preparations with demonstrated equal or equivalent reactions in the test.

It should be apparent to those skilled in the art that many more modifications besides those already described are possible without departing from the concepts herein. The subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. A method of using serum from (i) patients diagnosed with or suspected to have osteoarthritis and (ii) healthy control group individuals to identify whether a food is suitable for inclusion into a food sensitivity panel for testing food sensitivity in patients where the patient is diagnosed with or suspected to have osteoarthritis, the method comprising:
(a) immobilising food antigen preparations onto respective microtiter wells,
(b) allowing the food antigens to react with antibodies present in the patients' serum to form an antigen-antibody complex,
(c) removing excess serum proteins by a wash step,
(d) detecting IgG antibody binding by allowing an enzyme labeled anti-IgG antibody conjugate to react with the antigen-antibody complex,
(e) developing a color by adding a substrate that reacts with the coupled enzyme,
(f) measuring color intensity to obtain a signal score for each food antigen preparation,
(g) modeling signal scores against gender using a two-sample t-test and storing the residuals for further analysis, and
(h) comparing residual signal scores between osteoarthritis and controls using a permutation test on a two-sample t-test using the Satterthwaite approximation for the denominator degrees of freedom, to obtain a 2-tailed permuted p-value that represents a raw p-value for the food,
wherein a p-value of ≤ 0.07 as determined by raw p-value indicates the food is suitable for inclusion into the food sensitivity panel.

2. The method of claim 1, further comprising adjusting false discovery rates among the comparisons by any acceptable statistical procedures, typically using a statistical procedure selected from the group consisting of Benjamini-Hochberg, Family-wise Error Rate (FWER), and Per Comparison Error Rate (PCER), to obtain a 2-tailed FDR multiplicity-adjusted p-value that represents an FDR multiplicity adjusted p-value for the food, wherein a p-value of ≤ 0.10 as determined by FDR multiplicity adjusted p-value indicates the food is suitable for inclusion into the food sensitivity panel.

3. A method of making a food sensitivity panel for testing food sensitivity in patients where the patient is diagnosed with or suspected to have osteoarthritis, the method comprising coupling a plurality of distinct food preparations to individually addressable respective solid carriers, wherein the distinct food preparations have a discriminatory p-value of ≤ 0.07 as determined by raw p-value or a discriminatory p-value of ≤ 0.10 as determined by FDR multiplicity adjusted p-value when the food is tested by the method of claim 1 or 2.

4. A test panel obtainable by the method of claim 3.

5. The test panel of claim 4, wherein the solid carrier is a well of a multiwall plate, a bead, an electrical sensor, a chemical sensor, a microchip or an adsorptive film.

6. The test panel of claim 4, wherein the solid carrier is in the form of an array or a micro well plate.

7. The test panel of any one of claims 4-6, wherein the plurality of distinct food preparations is selected from a group consisting of chocolate, grapefruit, honey, malt, rye, baker's yeast, brewer's yeast, broccoli, cola nut, tobacco, mustard, green pepper, buck wheat, avocado, cane sugar, cantaloupe, garlic, cucumber, cauliflower, sunflower seed, lemon, strawberry, eggplant, wheat, olive, and optionally from a group consisting of halibut, cabbage, orange, rice, safflower, tomato, almond, oat, barley, peach, grape, potato, spinach, sole, and butter.

8. A method of testing food sensitivity in patients that are diagnosed with or suspected to have osteoarthritis using the test panel of any one of claims 4-7, the method comprising:
(a) contacting a food preparation with a bodily fluid of a patient that is diagnosed with or suspected to have osteoarthritis, wherein the bodily fluid is associated with a gender identification, and wherein the step of contacting is performed under conditions that allow immunoglobulin from the bodily fluid to bind to at least one component of the food preparation,
(b) quantifying/measuring the immunoglobulin bound to the component(s) of the food preparation to obtain a signal for each food preparation,
(c) comparing the signal against a gender-stratified reference value for the food preparation using the gender identification to obtain a result, and
(d) using the result to update or generate a report,
wherein if the observed signal is equal or more than the cutpoint value, then it is determined "positive" food, and wherein if the observed signal is less than the cutpoint value, then it is determined "negative" food.

9. A method of using the test panel of any one of claims 4-7 to confirm whether food sensitivities underlie a patient's symptoms of osteoarthritis, the method comprising:
(a) testing a body fluid sample of an osteoarthritis patient with an ELISA test using a test panel with 24 food samples using the method of claim 8,
(b) counting the number of foods that a patient tests positive for,
wherein the number of positive foods indicates whether it is likely that food sensitivities underlies on the patient's signs and symptoms of osteoarthritis,
wherein, if the patient is female and the reference value is a 90^{th} percentile value, then the number of positive foods used as a cutoff is 6 or 7,
wherein, if the patient is male and the reference value is a 90^{th} percentile value, then the number of positive foods used as a cutoff is 4,
wherein, if the patient is female and the reference value is a 95^{th} percentile value, then the number of positive foods used as a cutoff is 4 or 5, and
wherein, if the patient is male and the reference value is a 95^{th} percentile value, then the number of positive foods used as a cutoff is 3 or 4.
